# EUROPEAN PATENT APPLICATION

(11) **EP 1 992 667 A1**
(43) Date of publication of application: **19.11.2008**
(21) Application number: 07290584.7
(22) Date of filing: 09.05.2007
(51) Int. Cl.: C09B 29/00, C09B 29/36, C09B 69/02, C09B 69/04, G11B 7/246, C07D 213/85

(54) **Pyridinone based azo dyes and their metal complex salts**

(71) Applicant: Clariant International Ltd., 4132 Muttenz (CH)
(72) Inventor: Lücke, Lars, 65719 Hofheim am Taunus (DE); Klein, Cédric, 67170 Brumath (FR); Graciet, Jean-Christophe, 68128 Village-Neuf (FR); Winter, Martin, Alexander, 79400 Kandern (DE)

(57) **Abstract**

The present invention relates to pyridinone based azo dyes and/or of anionic azo metal complex dye salts made thereof with cationic basic yellow dyes, which are characterized by an unsaturated bond in beta-position to the endocyclic N atom of the pyridinone, and to their use in optical layers for optical data recording, preferably for optical data recording using a laser with a wavelength up to 450 nm.

The invention further relates to a write once read many (WORM) type optical data recording medium capable of recording and reproducing information with radiation of blue laser, which employs pyridinone based azo dyes and/or anionic azo metal complex dye salts made thereof with cationic basic yellow dyes, which are characterized by an unsaturated bond in beta-position to the endocyclic N atom of the pyridinone, in the optical layer.

## Description

The present invention relates to pyridinone based azo dyes and/or of anionic azo metal complex dye salts made thereof with cationic basic yellow dyes, which are characterized by an unsaturated bond in beta-position to the endocyclic N atom of the pyridinone, and to their use in optical layers for optical data recording, preferably for optical data recording using a laser with a wavelength up to 450 nm.

The invention further relates to a write once read many (WORM) type optical data recording medium capable of recording and reproducing information with radiation of blue laser, which employs pyridinone based azo dyes and/or anionic azo metal complex dye salts made thereof with cationic basic yellow dyes, which are characterized by an unsaturated bond in beta-position to the endocyclic N atom of the pyridinone, in the optical layer.

Recently, organic dyes have attracted considerable attentions in the field of diode-laser optical data storage. WORM type optical data recording media like commercial recordable compact discs (CD-R) and recordable digital versatile discs (DVD-R) can contain in the recording layer dyes based on phthalocyanine, hemicyanine, cyanine and metallized azo structures. These dyes are suitable in their respective fields with the laser wavelength criteria. Other general requirements for dye media are strong absorption, high reflectance, high recording sensitivity, enhancement of photosensitivity, low thermal conductivity as well as light and thermal stabilities, durability for storage or non-toxicity. Important criteria are also good read-out stability, which means high number of cycles at a given intensity of laser-light, and sufficient solubilities of the dyes in the organic solvents generally applied in the spin coating process.

At the recorded region of such an organic dye type optical data recording medium, the optical properties have been changed not only by a change in the optical characteristics and a decrease in the layer thickness resulting from the thermal decomposition of the dye, but also by a deformation of the substrate.

This recording principle is the same for CD-R and DVD-R, the difference remaining the spot size and the wavelength of the laser light used. CD-R are writable at a wavelength of from 770 to 830 nm and DVD-R, by using more recent compact high-performance red diode lasers, at a wavelength from 600 to 700 nm achieving then a 6- to 8 fold improvement in data packing density in comparison with conventional CDs.

However, considering factors such as the recent spread of electronic networks (e.g. Internet) and the emergence of high definition television (HDTV) broadcasting, inexpensive and convenient recording media, capable of recording image information at even larger capacity, are required. While DVD-R's sufficiently serve as high-capacity recording media at present, demand for larger capacity and higher density has increased.

Blu-ray® discs (Blu-ray® disc is a standard developed by Hitachi Ltd., LG Electronics Inc., Matsushita Electric Industrial Co. Ltd., Pioneer Corporation, Royal Philips Electronics, Samsung Electronics Co. Ltd., Sharp Corporation, Sony Corporation, Thomson Multimedia) or HD-DVD discs (a standard developed by Toshiba and NEC) are going to be the next milestone in optical data recording technology. By these new specifications the data storage may be increased up to 27 Gigabytes per recording layer for a 12 cm diameter disc. By adopting a blue diode laser with a wavelength of 405 nm (GaN or SHG laser diodes), the pit size and track interval can be further reduced, again increasing the storage capacity by an order of magnitude.

The construction of such optical data recording media is known in the art. The optical recording medium comprises preferably a substrate with a guide groove for laser beam tracking, a recording layer, this recording layer also being called optical layer or dye layer in the following text, containing an organic dye as the main component, a reflective layer and a protective layer. When recording/readout is carried out through the substrate, a transparent substrate is employed. As such a transparent substrate, one made of a resin such as polycarbonate, polymethacrylate or amorphous polyolefin, one made of glass or one having a resin layer made of radiation curable resin, i.e. photopolymerizable resin, formed on glass, may, for example, be employed. Advanced optical data recording media may comprise further layers, such as protective layers, adhesive layers or even additional optical recording layers.

For blue diode-laser optical data storage a variety of dye compounds has been proposed in the literature.

WO 2006/106110 A discloses anionic azo metal complex dyes with cationic basic yellow dyes as counterion.

Unfortunately the dye compounds described so far still show disadvantages which impede their satisfactory use as dyes for optical data storage.

There is a still a need for an optical data recording medium that is capable of recording data at high density with improved recording characteristics and with improved read-out stabilities, also for the recording at speeds exceeding 1X, i.e. for 2X speed and 4X speed recording, therefore a need for an optical data recording medium with improved recording characteristics.

Surprisingly the object was achieved by using pyridinone based azo dyes and/or of anionic azo metal complex dye salts made thereof with cationic basic yellow dyes, which are characterized by an unsaturated bond in beta-position to the endocyclic N atom of the pyridinone.

In the following text, "halogen" represents F, Cl, Br or I, preferably F, Cl or Br, more preferably F or Cl, even more preferably Cl, if not otherwise stated; "alkyl" represents linear and branched alkyl; and "alkoxy" represents linear and branched alkoxy; "alkenyl" represents linear and branched alkenyl, "alkynyl" represents linear and branched alkynyl, any alkyl and cycloalkyl groups being unsubstituted, partially or completely substituted by halogen; an "alkenyl" residue has at least one, or one or more, and at most as many as possible double bonds; an "alkynyl" residue has at least one, or one or more, and at most as many as possible triple bonds; if not otherwise stated.

Subject of the invention is a compound of formula (I), 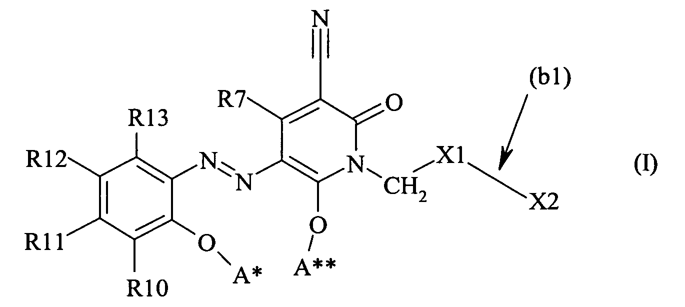 wherein the residues A* and A**each represent H
or the residues A* and A** together represent a group of formula (II); 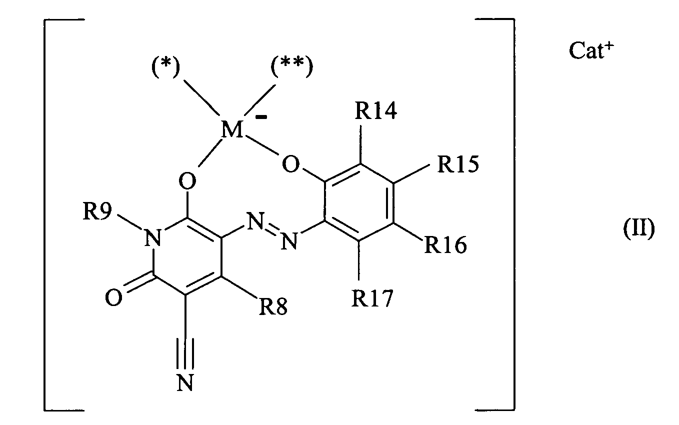
- M: represents a trivalent metal atom, preferably selected from groups 3, 4, 5, 6, 7, 8, 9, 10, 11 and 12 of the Periodic Table of the Chemical Elements;
the (*) in formula (II) indicating the bond from the metal atom M to the O atom, which is connected to the residue A* in formula (I), and
the (**) in formula (II) indicating the bond from the metal atom M to the O atom, which is connected to the residue A** in formula (I); the bond (b1) between X1 and X2 in formula (I) being an unsaturated bond selected from the group consisting of a double bond, a triple bond and an aromatic bond;
- X1: represents a carbon atom in the case, that the bond (b1) to X2 is a triple bond, or
- X1: represents -CH- in the case, that the bond (b1) to X2 is a double bond;
- X2: represents a N atom or CH in the case, that the bond (b1) to X2 is a triple bond, or
- X2: represents CH2 in the case, that the bond (b1) to X2 is a double bond; or
- X1: together with X2 form a phenyl group and the bond (b1) is an aromatic bond;

- Cat⁺: is a cation selected from the group consisting of basic yellow cations of Basic Yellow dyes, compounds of formula (a) and compounds of formula (g); 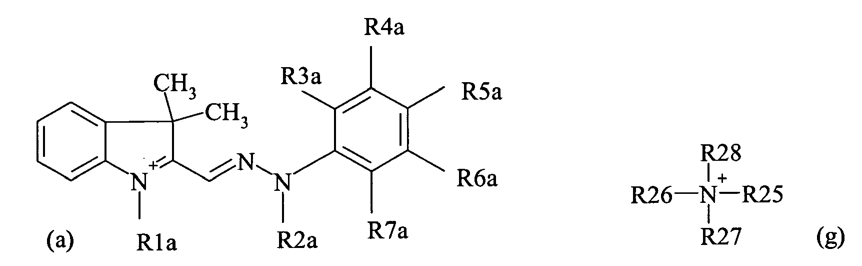
- R9: is selected from the group consisting of -C₁₋₄ alkyl, -NH-phenyl, -CH₂-CH=CH₂, -CH₂-C≡CH, -CH₂-CN and benzyl;
- R7 and R8: are identical or different and independently from each other selected from the group consisting of H, CN, CF₃, halogen, NO₂, OH, SH, SO₂-NR²¹R²², CO-R²⁰, SO₂R²⁰, CO-NR²¹R²²,
C₁₋₁₀ alkyl, C₃₋₁₀ cycloalkyl, the C₁₋₁₀ alkyl and the C₃₋₁₀ cycloalkyl being independently from each other unsubstituted or substituted by 1 to 4 identical or different substituents, the substituents being independently from each other selected from the group consisting of C₁₋₁₀ alkyl, halogen, OH, C₆₋₁₂ aryl and NR²¹R²²,
C₆-C₁₂ aryl the C₆₋₁₂ aryl being unsubstituted or substituted by 1 to 4 identical or different substituents, the substituents being independently from each other selected from the group consisting of C₁₋₁₀ alkyl, C₃₋₁₀ cycloalkyl, OH, NO₂, CN, halogen, CF₃, C₆₋₁₂ aryl, C₁₋₁₀ alkoxy and NR²¹R²²,
OC₁₋₁₀ alkyl, NR²¹R²² and SC₁₋₁₀ alkyl;
- R3a, R4a, R5a, R6a, R7a, R10, R11, R12, R13, R14, R15, R16 and R17: are identical or different and independently from each other selected from the group consisting of H, CN, CF₃, halogen, NO₂, OH, SH, SO₂-NR²¹R²², CO-R²⁰, SO₂R²⁰, CO-NR²¹R²²,
C₁₋₁₀ alkyl, C₃₋₁₀ cycloalkyl, the C₁₋₁₀ alkyl and the C₃₋₁₀ cycloalkyl being independently from each other unsubstituted or substituted by 1 to 4 identical or different substituents, the substituents being independently from each other selected from the group consisting of C₁₋₁₀ alkyl, halogen, OH, CN, CF₃, C₆₋₁₂ aryl and NR²¹R²²,
C₆-C₁₂ aryl, O-C₆₋₁₂ aryl, S-C₆₋₁₂ aryl, the C₆₋₁₂ aryl and the O-C₆₋₁₂ aryl and the S-C₆₋₁₂ aryl being unsubstituted or substituted by 1 to 4 identical or different substituents, the substituents being independently from each other selected from the group consisting of C₁₋₁₀ alkyl, C₃₋₁₀ cycloalkyl, OH, NO₂, CN, halogen, CF₃, C₆₋₁₂ aryl, O-C₁₋₁₀ alkyl, S-C₁₋₁₀ alkyl and NR²¹R²²,
OC₁₋₁₀ alkyl, SC₁₋₁₀ alkyl, O-C₃₋₁₀ cycloalkyl, S-C₃₋₁₀ cycloalkyl, NHCOR²⁰ and NR²¹R²²;
- the R²¹ and R²²: residues are identical or different and independently from each other selected from the group consisting of H, C₁₋₁₀ alkyl, C₆₋₁₂ aryl and C₁₋₁₂ alkyl-NR²³R²⁴;
- the R²³ and R²⁴: residues are identical or different and independently from each other selected from the group consisting of H, C₁₋₁₀ alkyl and C₆₋₁₂ aryl;
- the R²⁰: residues are identical or different and independently from each other selected from the group consisting of OH, C₁₋₆ alkyl, C₆₋₁₀ aryl and O-C₁₋₆ alkyl;
- R1a and R2a: are identical or different and independently from each other selected from the group consisting of
C₁₋₁₀ alkyl, C₂₋₁₀ alkenyl, C₂₋₁₀ alkynyl, C₃₋₁₀ cycloalkyl,
the C₁₋₁₀ alkyl, the C₂₋₁₀ alkenyl, the C₂₋₁₀ alkynyl and the C₃₋₁₀ cycloalkyl being independently from each other unsubstituted or substituted by identical or different substituents, preferably by 1 or more than 1 substituent, more preferably by 1, 2, 3 or 4 substituents, even more preferably by 1 substituent, the substituents being independently from each other selected from the group consisting of C₁₋₁₀ alkyl, halogen, CN, OH, CF₃, C₆₋₁₂ aryl, the C₆₋₁₂ aryl being unsubstituted or substituted by 1 to 5, preferably 1 to 3, more preferably 1 or 2, identical or different substituents, the substituents being independently from each other selected from the group consisting of C₁₋₁₀ alkyl, OH, NO₂, CN, halogen, CF₃, C₆₋₁₂ aryl, O-C₁₋₁₀ alkyl and S-C₁₋₁₀ alkyl;
the C₂₋₁₀ alkenyl having 1, 2 or 3, preferably 1 or 2, more preferably 1 double bond, the double bond being preferably a beta-gamma-double bond, more preferably the C₂₋₁₀ alkenyl represents allyl;
the C₂₋₁₀ alkynyl having 1, 2 or 3, preferably 1 or 2, more preferably 1 triple bond, the triple bond being preferably a beta-gamma-triple bond, more preferably the C₂₋₁₀ alkynyl represents propargyl;
the compounds of formula (g) being also called ammonium type cations of formula (g);
- R²⁵, R²⁶, R²⁷ and R²⁸: are identical or different and independently from each other selected from the group consisting of H, C₁₋₃₀ aliphatic hydrocarbon and C₁₋₃₀ aromatic hydrocarbon, the C₁₋₃₀ aliphatic and/or C₁₋₃₀ aromatic hydrocarbon being unsubstituted or substituted by 1 to 8 identical or different substituents independently from each other selected from the group consisting of halogen, C₁₋₁₀-alkyl, O-C₁₋₁₀-alkyl, CN, NH₂, OH, NO₂;
or where the substituents
- R²⁵ and R²⁶: together with the nitrogen atom of the ammonium ion of formula (g) to which they are attached
can form a five- to seven-membered saturated or unsaturated ring which can contain 1, 2 or 3, preferably 1 or 2 further identical or different heteroatoms independently from each other selected from the group consisting of O, S and N or carbonyl groups,
and which can carry 1 or 2 fused-on saturated, unsaturated or aromatic, carbocyclic or heterocyclic rings;
the ring and the rings fused on, where appropriate, can be substituted by 1, 2 or 3 identical or different radicals independently from each other selected from the group consisting of OH, NH₂, phenyl, CN, Cl, Br, C₁-C₄ alkyl and C₁-C₄ alkoxy,
preferably of the pyrrolidine, pyrrolidone, imidazolidine, hexamethyleneimine, piperidine, piperazine or morpholine type, more preferably of the pyrrolidine or morpholine type;
or where the substituents
- R²⁵, R²⁶ and R²⁷: together with the nitrogen atom of the ammonium ion of formula (g) to which they are attached
can form a five- to seven-membered aromatic ring which can contain 1, 2 or 3, preferably 1 or 2 further identical or different heteroatoms independently from each other selected from the group O, S and N or carbonyl groups,
and which can carry 1 or 2 fused-on saturated, unsaturated or aromatic, carbocyclic or heterocyclic rings;
the ring and the rings fused on, where appropriate, can be substituted by 1, 2 or 3 identical or different radicals independently from each other selected from the group consisting of OH, NH₂, phenyl, CN, Cl, Br, C₁-C₄ alkyl and C₁-C₄ alkoxy,
preferably of the pyrrole, imidazole, pyridine, picoline, pyrazine, quinoline or isoquinoline type.

Preferably,
- M: is selected from the group consisting of Co, Cr, Fe and A1;
- R1a and R2a: are identical or different and independently from each other selected from the group consisting of
C₁₋₄ alkyl, C₃ alkenyl, C₃ alkynyl,
the C₁₋₄ alkyl, the C₃ alkenyl and the C₃ alkynyl being independently from each other unsubstituted or substituted by identical or different substituents, preferably by 1, 2 or 3 substituents, even more preferably by 1 substituent, the substituents being independently from each other selected from the group consisting of methyl, halogen, CN, phenyl, the phenyl being unsubstituted or substituted by 1 or 2, preferably 1, identical or different substituents, the substituents being independently from each other selected from the group consisting of C₁₋₁₀ alkyl, OH, NO₂, CN, halogen, CF₃, C₆₋₁₂ aryl, O-C₁₋₁₀ alkyl and S-C₁₋₁₀ alkyl;
preferably the C₃ alkenyl represents allyl;
preferably the C₃ alkynyl represents propargyl;
- R3a, R4a, R5a, R6a and R7a: are identical or different and independently from each other selected from the group consisting of H, CN, CF₃, halogen, NO₂, C₁₋₄ alkyl, the C₁₋₄ alkyl being unsubstituted or substituted by 1 or 2, preferably by 1, identical or different substituents, the substituents being independently from each other selected from the group consisting of C₁₋₄ alkyl, halogen, CN, phenyl,
phenyl, O-phenyl, S-phenyl, the phenyl and the O-phenyl and the S-phenyl being unsubstituted or substituted by 1 to 2, preferably 1, identical or different substituents, the substituents being independently from each other selected from the group consisting of C₁₋₄ alkyl, halogen, O-C₁₋₄ alkyl and S-C₁₋₄ alkyl,
O-C₁₋₄ alkyl, S-C₁₋₄ alkyl and NR²¹R²²;
- the R²¹ and R²²: residues are identical or different and independently from each other selected from the group consisting of H and C₁₋₄ alkyl.

More preferably,
- M: is selected from the group consisting of Co, Fe and A1;
- R1a and R2a: are identical or different and independently from each other represent methyl or allyl;
- R3a, R4a, R5a, R6a and R7a: are identical or different and independently from each other selected from the group consisting of H, C₁₋₃ alkyl, preferably methyl, ethyl, n-propyl or iso-propyl, further O-phenyl, S-phenyl, methoxy and ethoxy.

Even more preferably,
- M: is selected from the group consisting of Co and Fe;
- R1a and R2a: are identical or different and independently from each other represent methyl or allyl;
- R3a, R4a, R6a and R7a: are identical or different and independently from each other selected from the group consisting of H, iso-propyl, S-phenyl and methoxy;
- R5a: is selected from the group consisting of H, iso-propyl and S-phenyl.

Preferably,
- R²⁵, R²⁶, R²¹ and R²⁸: independently from each other are selected from the group consisting of H,
dehydroabietyl radical, the dehydroabietyl radical derived from dehydroabietylamine, i.e. (4β)-abieta-8,11,13-trien-18-amine);
C₁₋₁₀ alkyl, more preferably C₁₋₄ alkyl, the C₁₋₁₀ alkyl and the C₁₋₄ alkyl being unsubstituted or substituted by halogen, C₁₋₄-alkyl, OH and CN,
unsubstituted or substituted phenyl, with 1 to 4 substituents independently from each other being selected from the group consisting of halogen, methoxy, ethoxy, C₁₋₁₀ alkyl and NO₂, more preferably of C₁₋₂ alkyl, and
unsubstituted or substituted benzyl, with 1 to 4 substituents independently from each other being selected from the group consisting of halogen, methoxy, ethoxy, C₁₋₁₀ alkyl and NO₂;
or where the substituents
- R²⁵ and R²⁶: together with the nitrogen atom of the ammonium ion of formula (g) to which they are attached form a ring of the pyrrolidine or morpholine type.

The basic yellow cations of Basic Yellow dyes, the expression Basic Yellow as defined in the Color Index: colour index international, fourth edition, © Society of Dyers and Colourists and American Association of Textile Chemists and Colorists 2002, are preferably selected from the group of cations consisting of the cations of Basic Yellow 1, Basic Yellow 2, Basic Yellow 11, Basic Yellow 13, Basic Yellow 21, Basic Yellow 24, Basic Yellow 28, Basic Yellow 29, Basic Yellow 37, Basic Yellow 49, Basic Yellow 51, Basic Yellow 57 and Basic Yellow 90.

More preferably,
subject of the invention is a compound of formula (I) with the residues A* and A** together representing a group of formula (II), in all the preferred aspects of the formula (I) with the residues A* and A** together representing a group of formula (II)as described above;
- R²⁵, R²⁶, R²⁷ and R²⁸: independently from each other are selected from the group consisting of H, methyl, ethyl, isopropyl, methoxy, ethoxy, n-butyl, -(CH₂)₂-CN, phenyl, tolyl, benzyl;
or where the substituents
- R²⁵: and R²⁶ together with the nitrogen atom of the ammonium ion of formula (g) to which they are attached, form a ring of the pyrrolidine or morpholine type;
the basic yellow cation is selected from the group of cations consisting of the cations of Basic Yellow 1, Basic Yellow 2, Basic Yellow 11, Basic Yellow 13, Basic Yellow 28 and Basic Yellow 29; 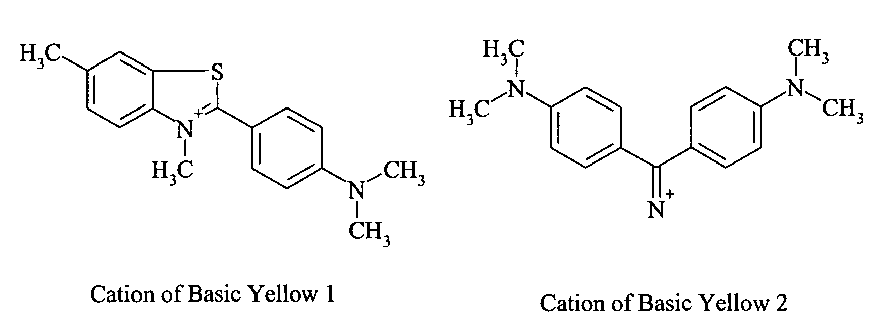 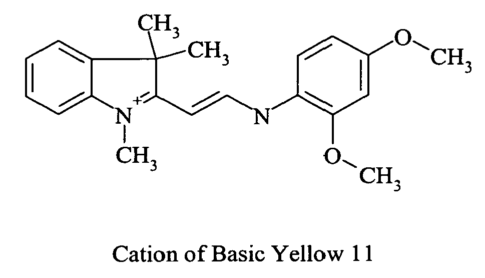 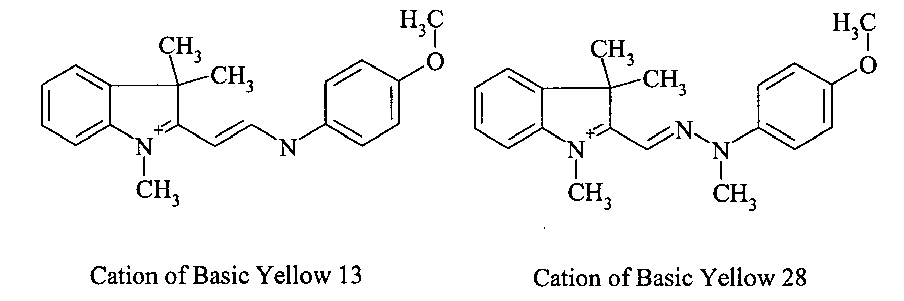 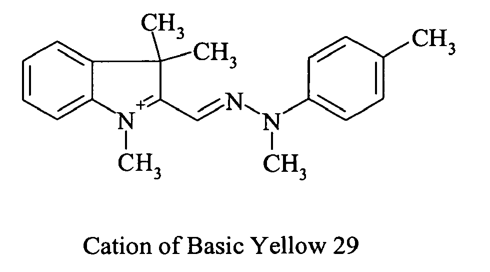 or a compound selected from the group of cations consisting of the modified Basic Yellow cations of formula a1, a2, a3, a4, a5, a6 and a7; 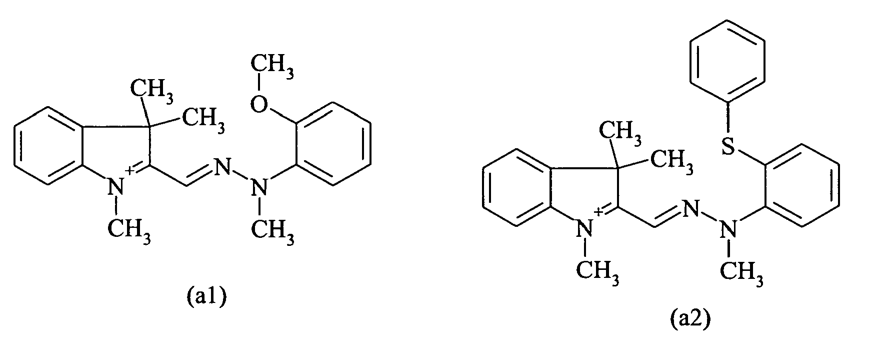 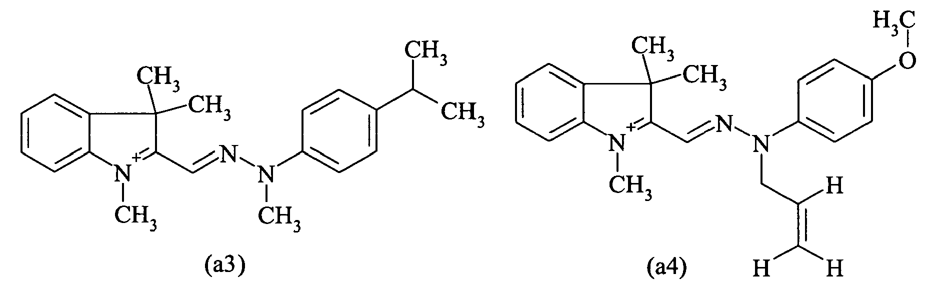 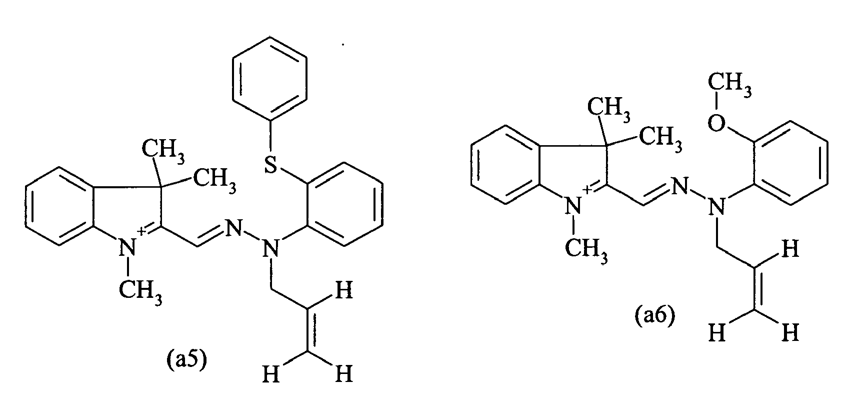 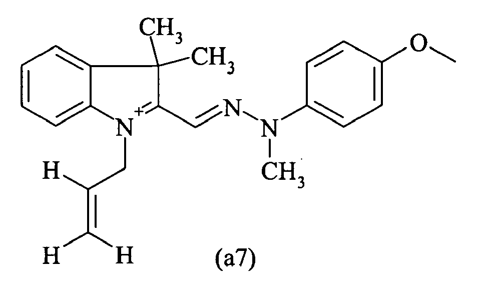 or the ammonium type cation of formula (g) is an ammonium type cation of formula (g1), (g2), (g3), (g4) or (g5), especially (g2).

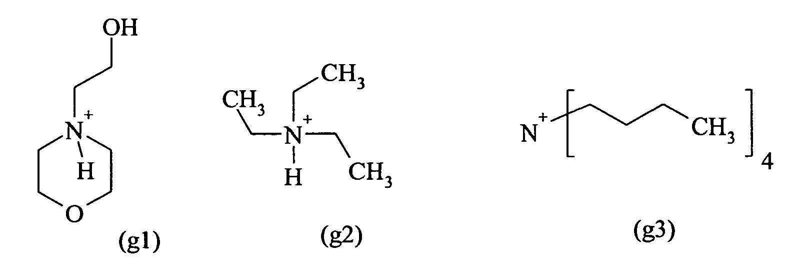

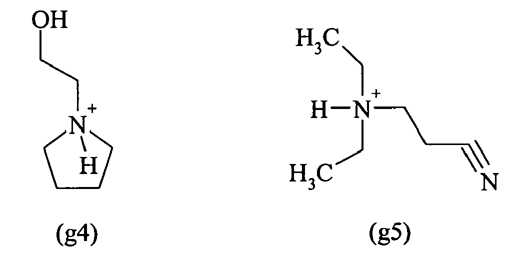

More especially, the compounds of formula (I) are selected from the group consisting of compounds of formulae (d1), (d2), (d3), (d4), (d5), (e1BY28), (e1a1), (e1a2), (e1a3), (e1a4), (e1a5), (e1a6), (e1a7), (e2), (e2BY28), (e2a2), (e3), (e3BY28), (e4), (e4BY28), (e4a2), (e5), (e5BY28), (e5a2), (e5a4), (e6), (e6BY28), (e6a2), (e7), (e7BY28), (e8), (e8BY28) and (e9);
even more especially from the group consisting of compounds of formulae (e1BY28), (e1a2), (e1a3), (e1a4), (e1a5), (e2BY28) and (e3BY28).

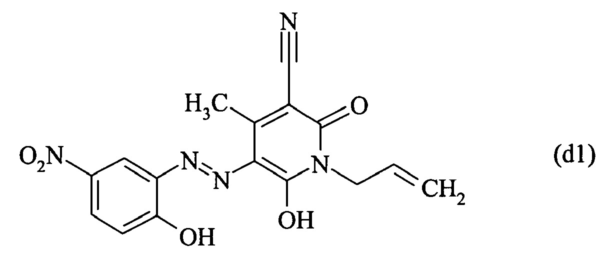

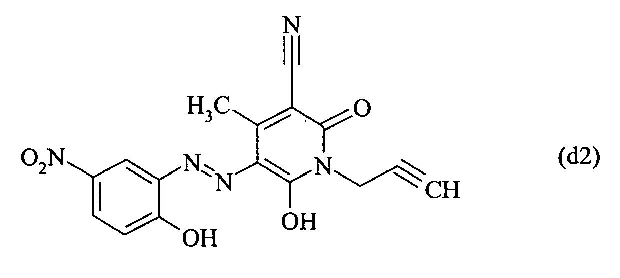

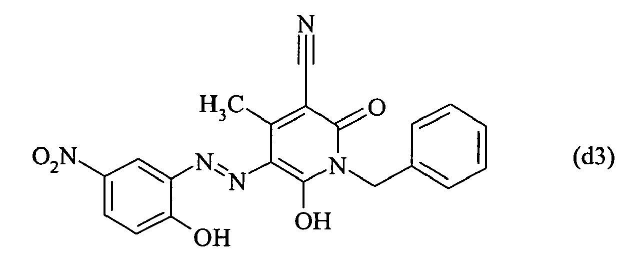

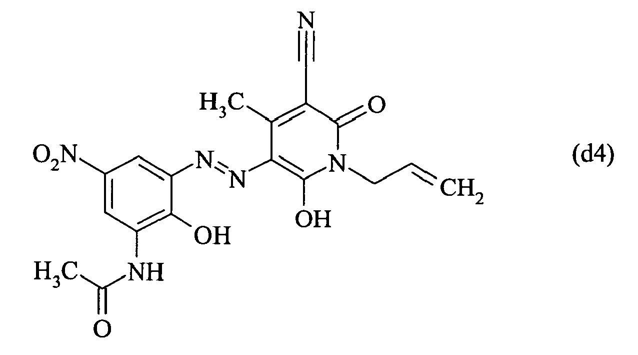

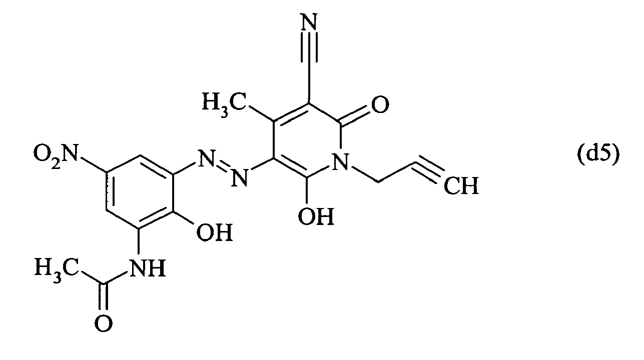

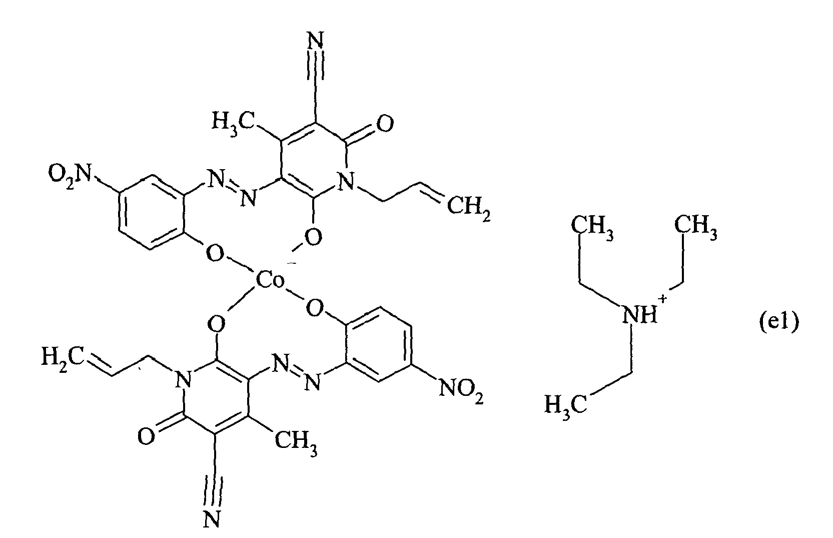

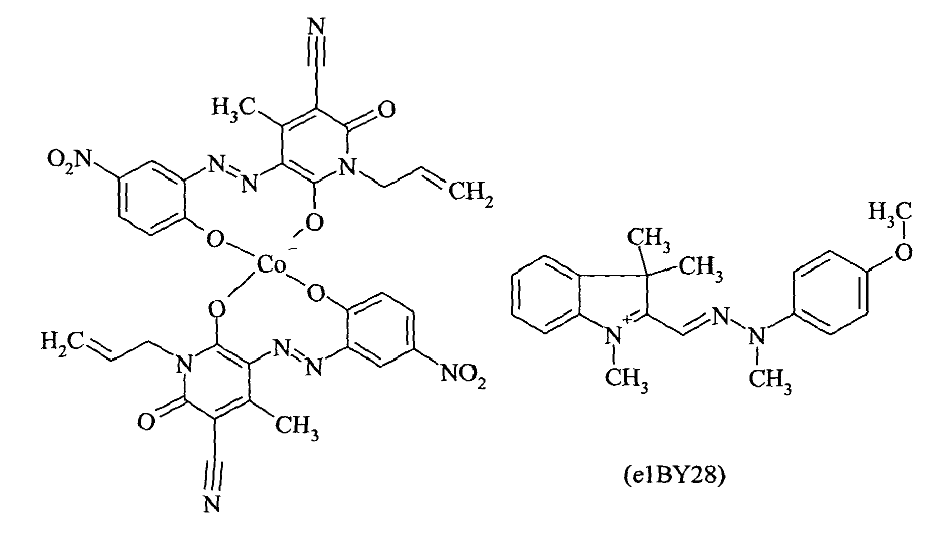

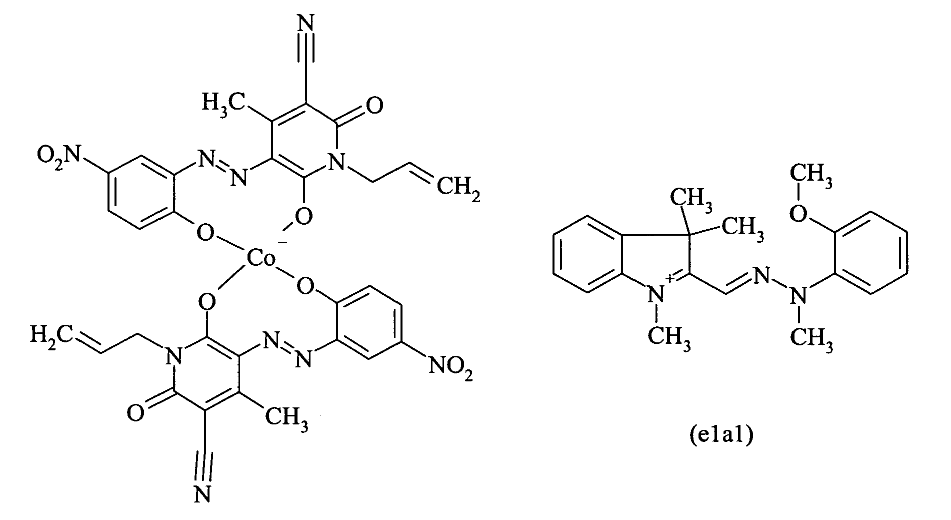

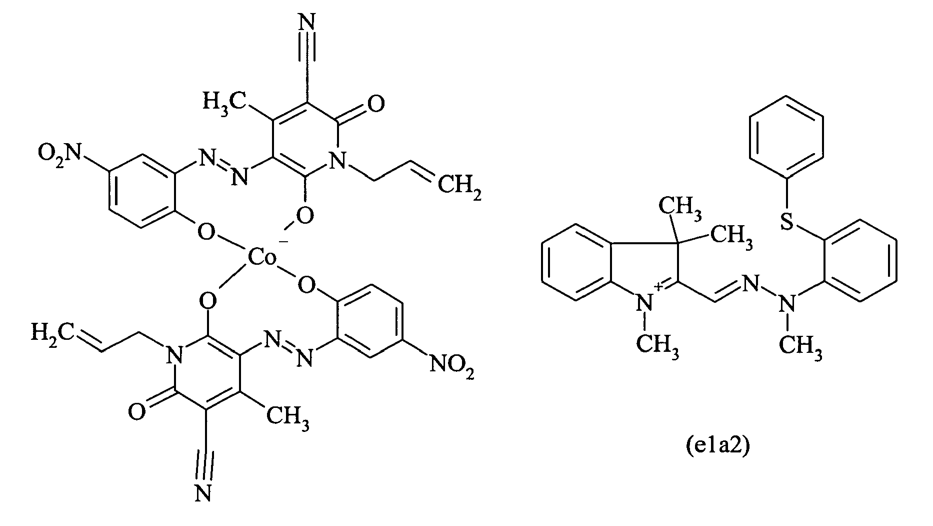

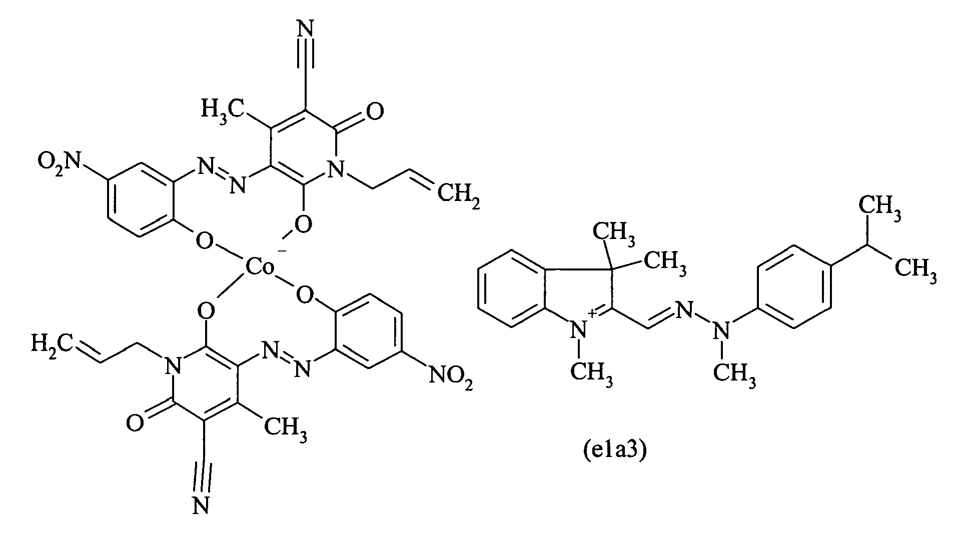

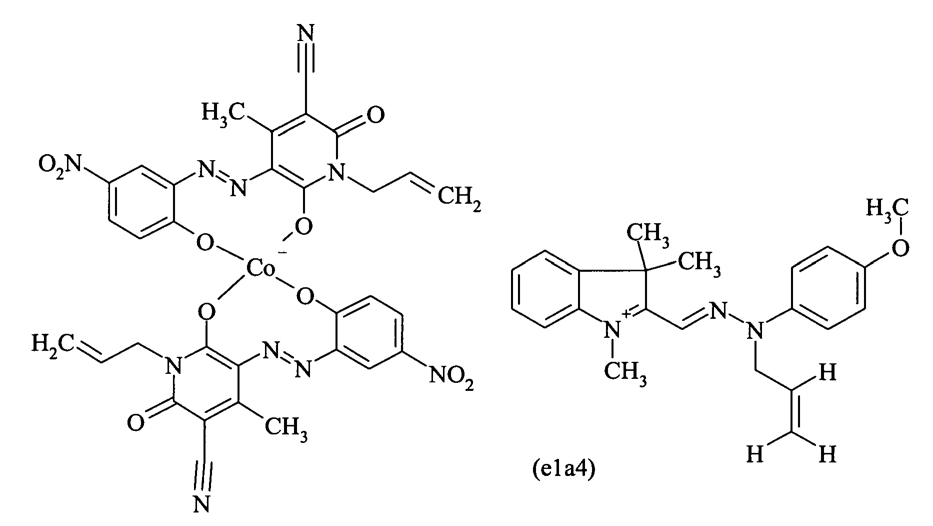

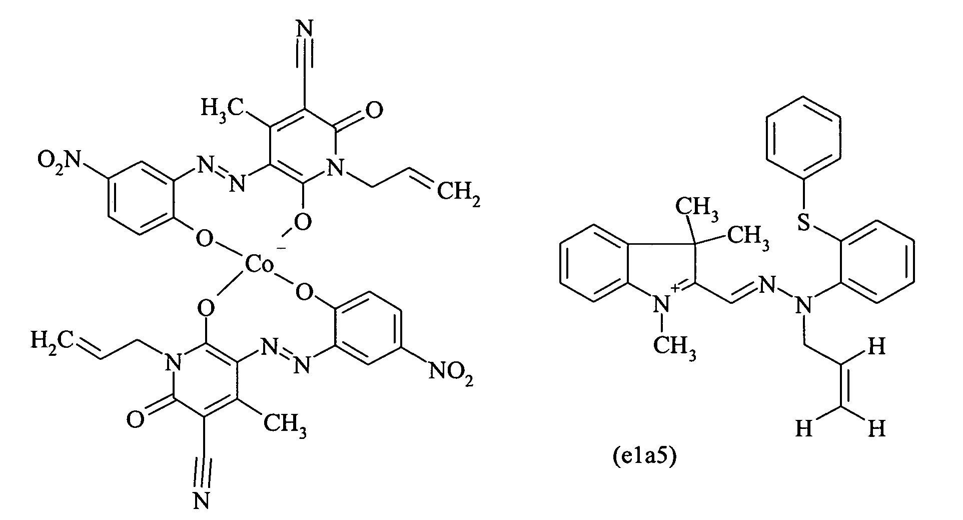

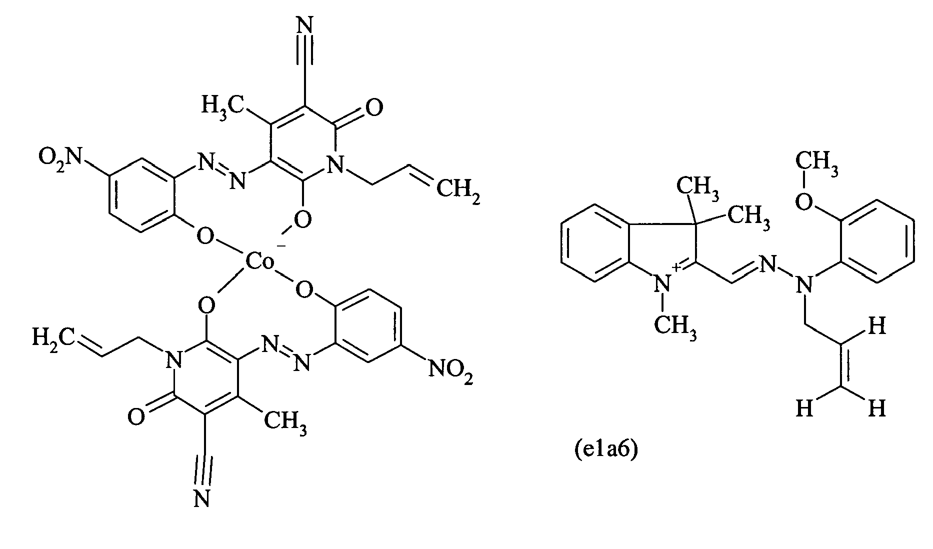

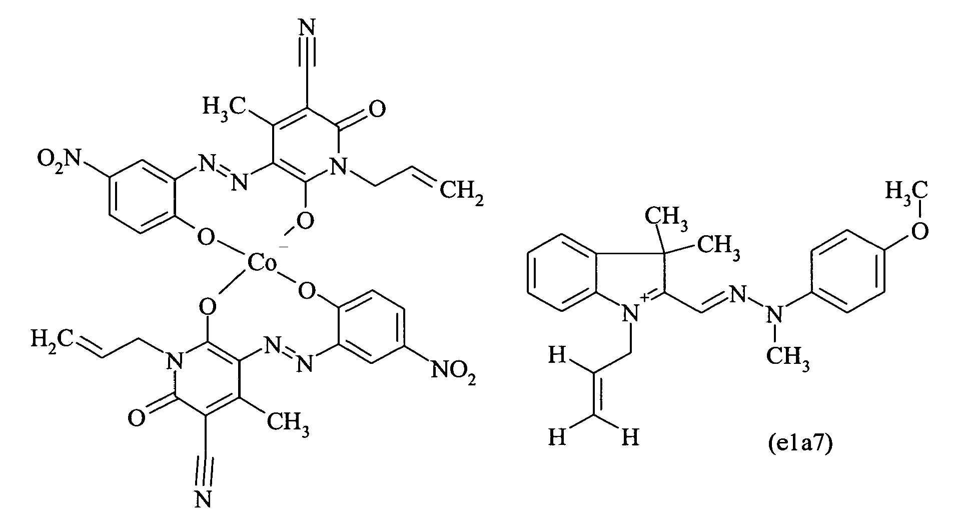

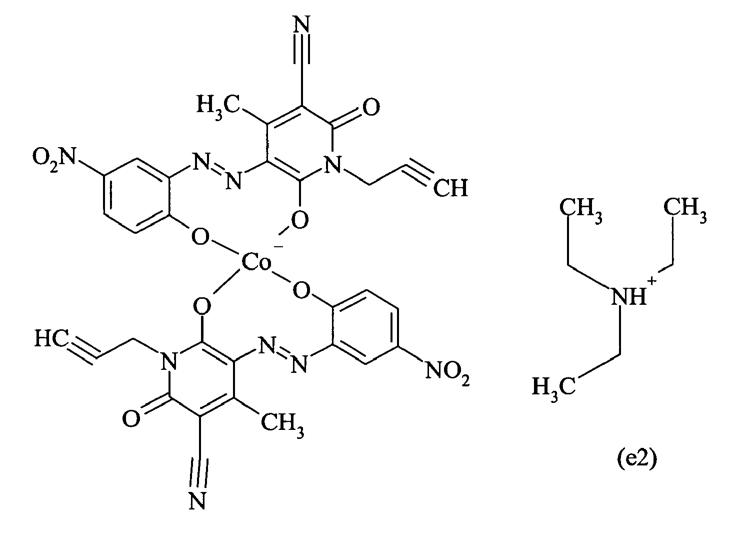

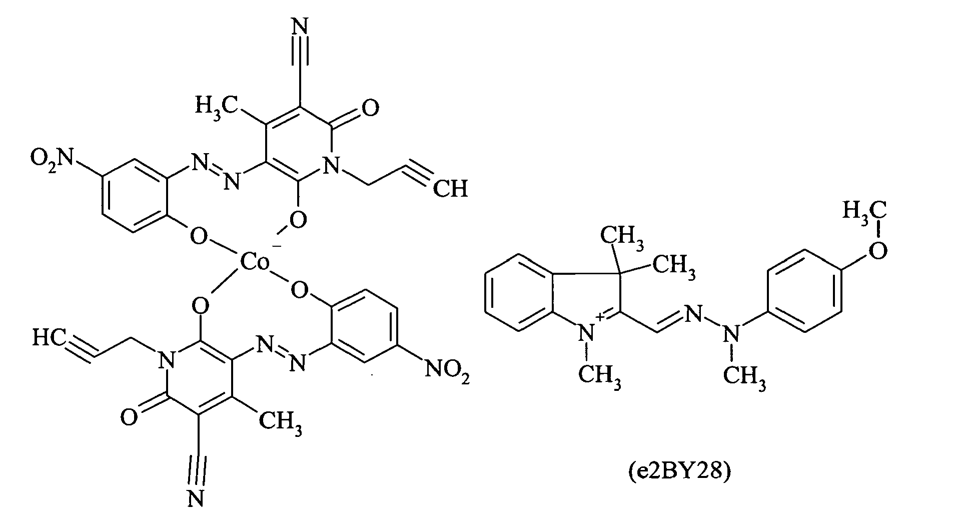

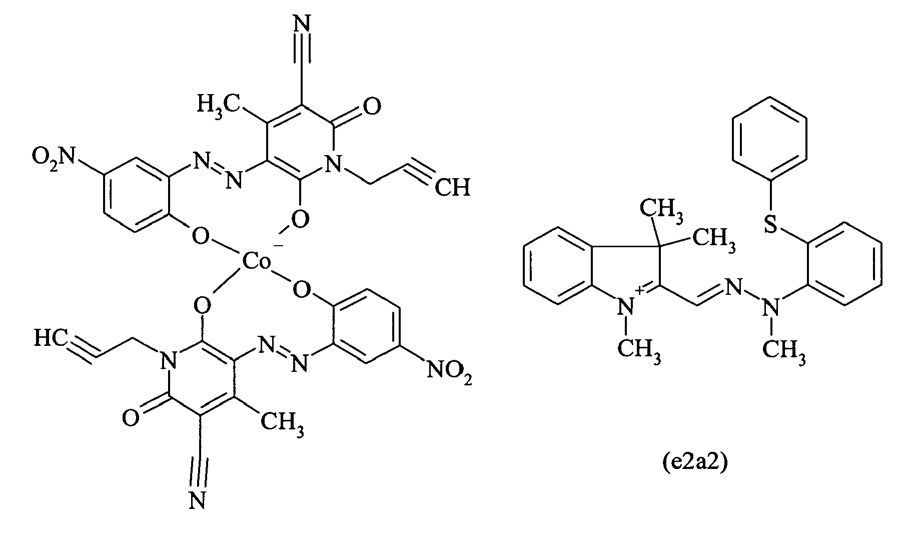

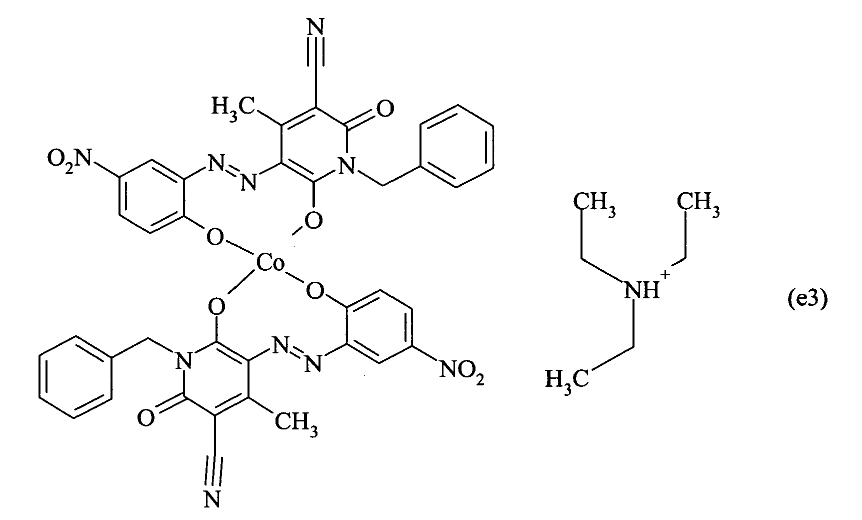

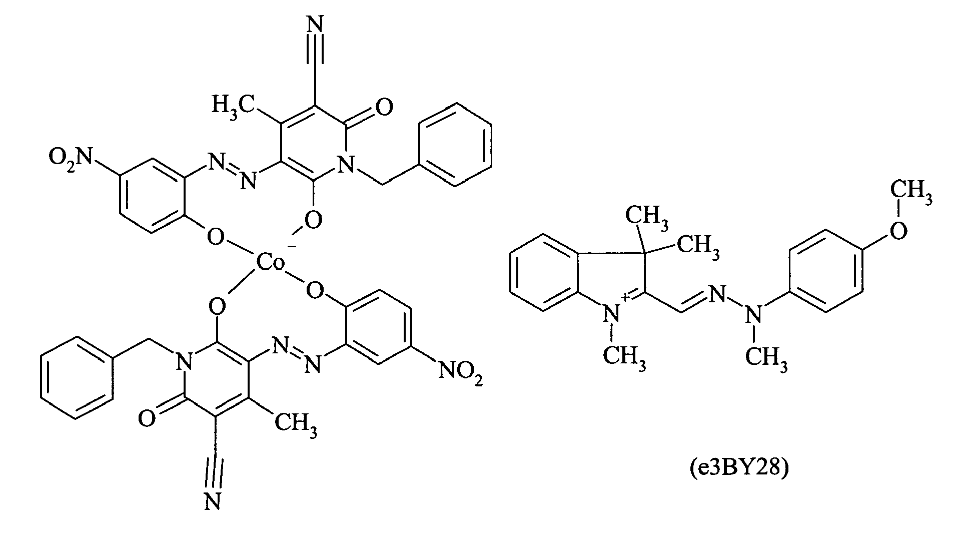

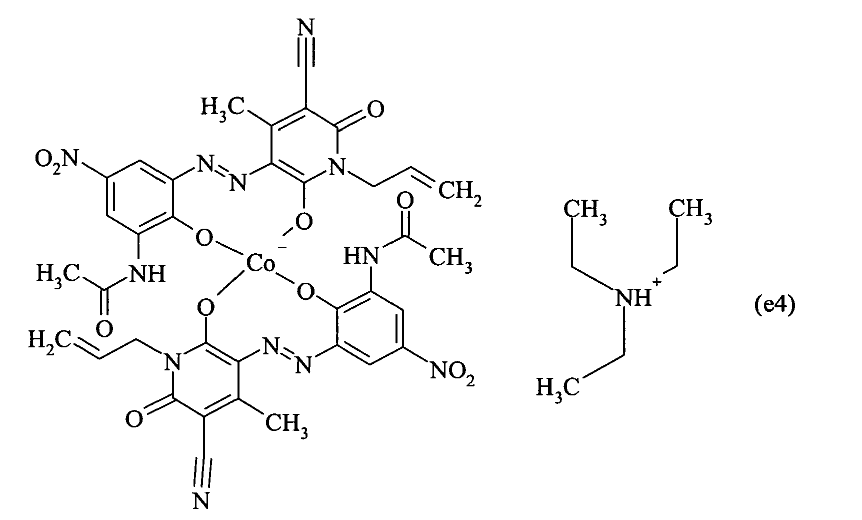

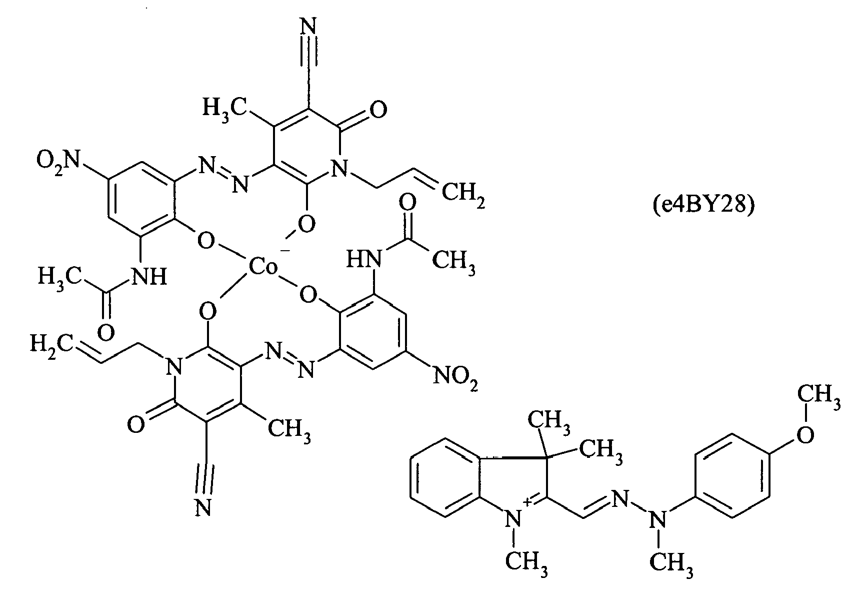

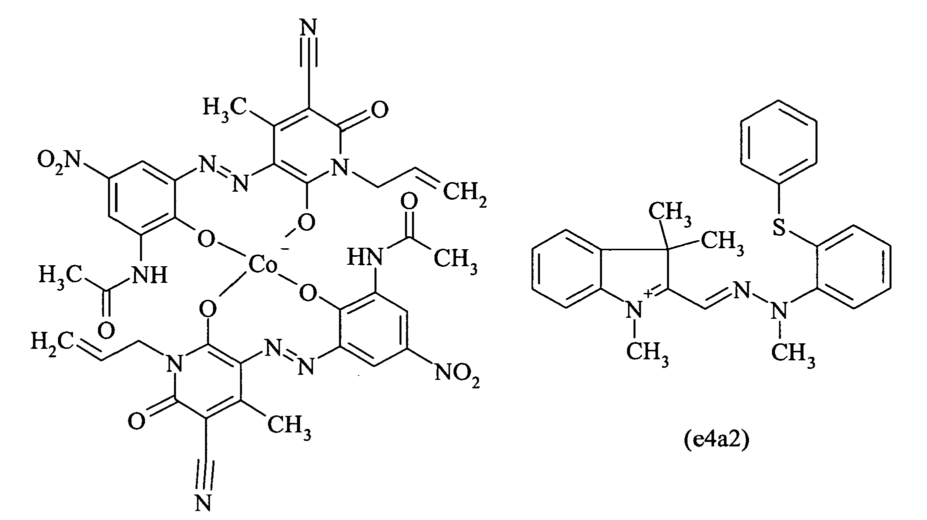

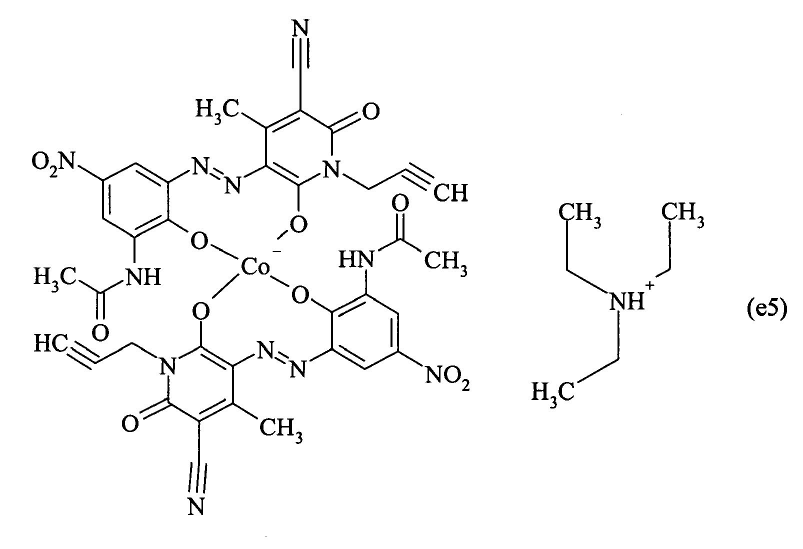

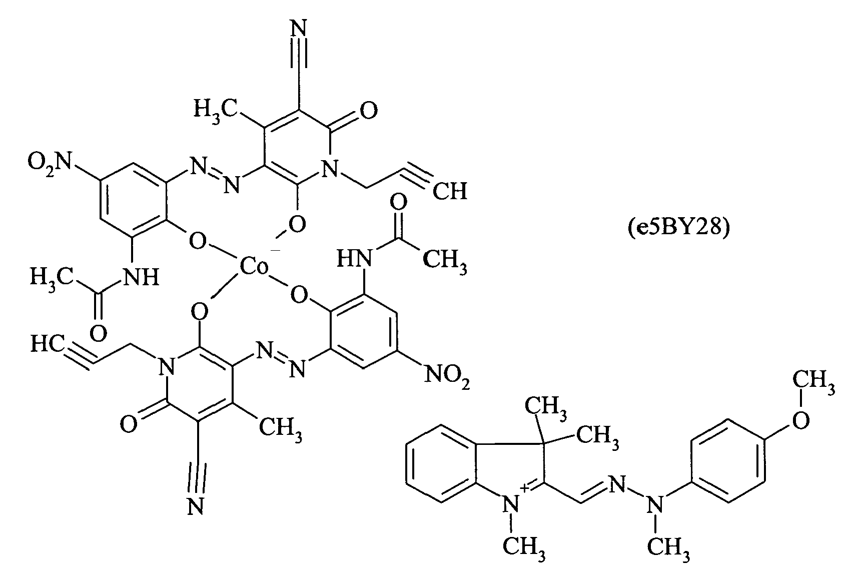

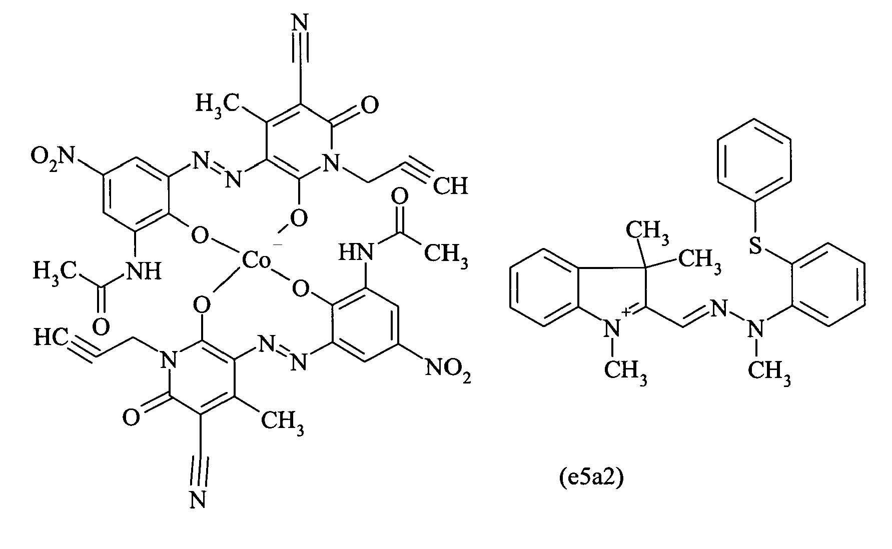

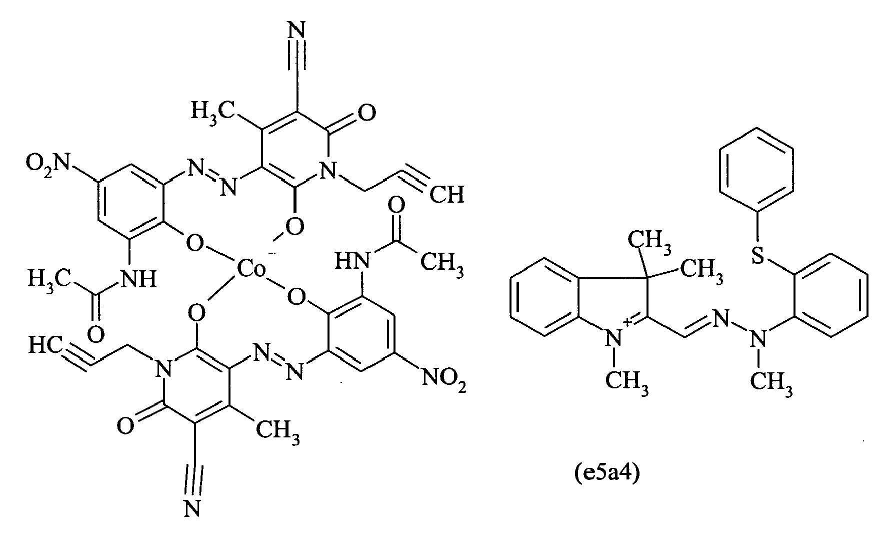

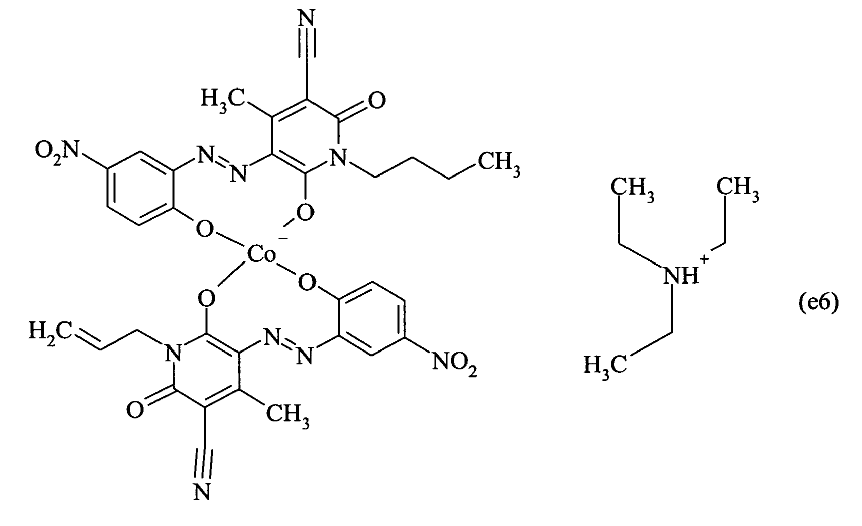

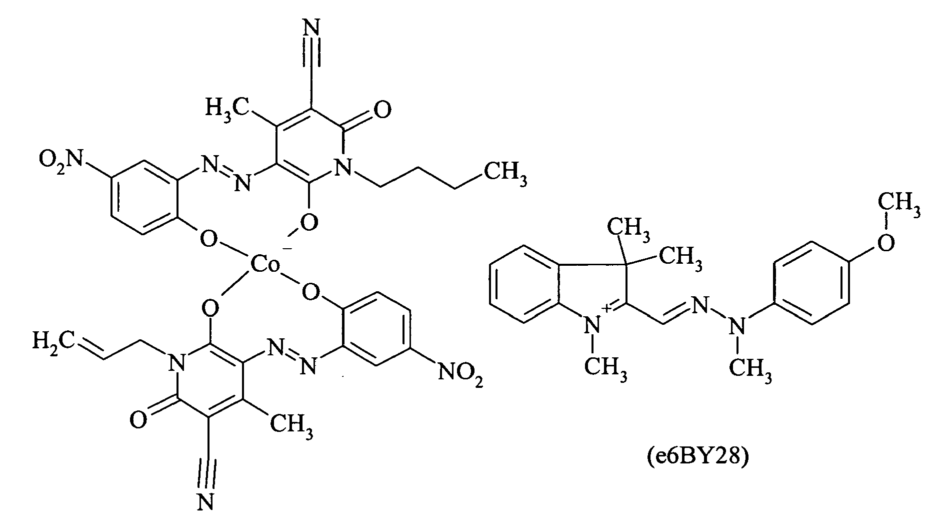

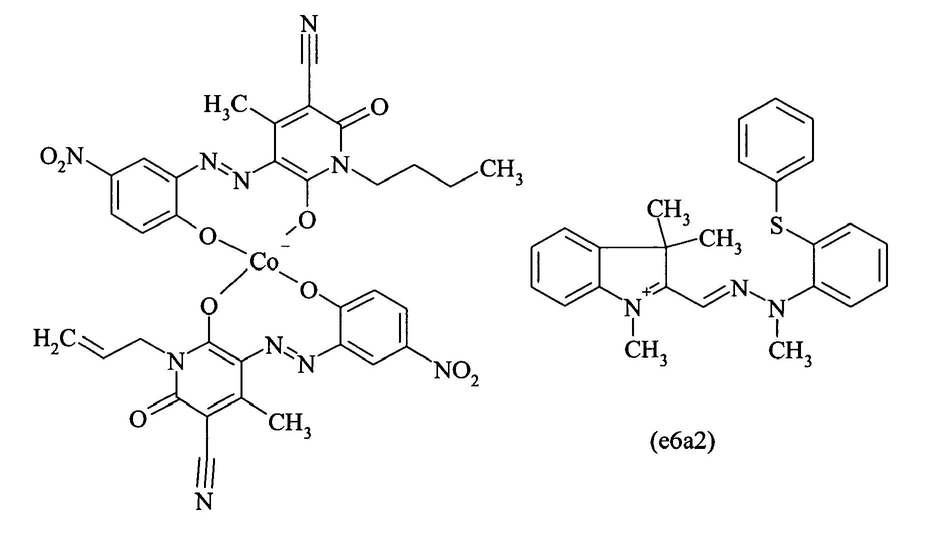

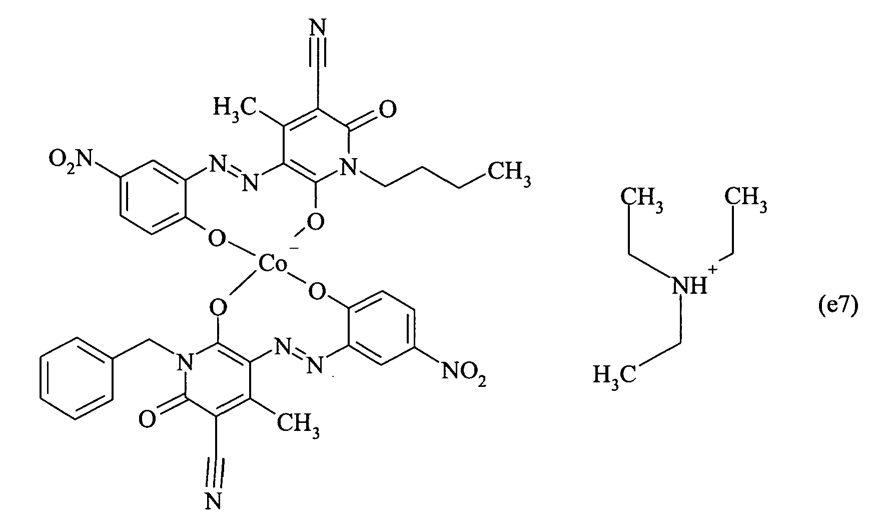

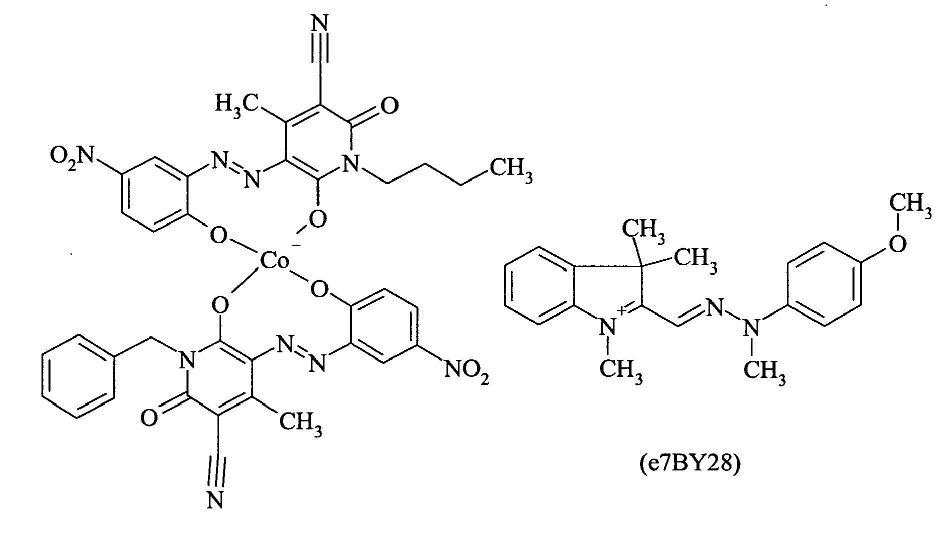

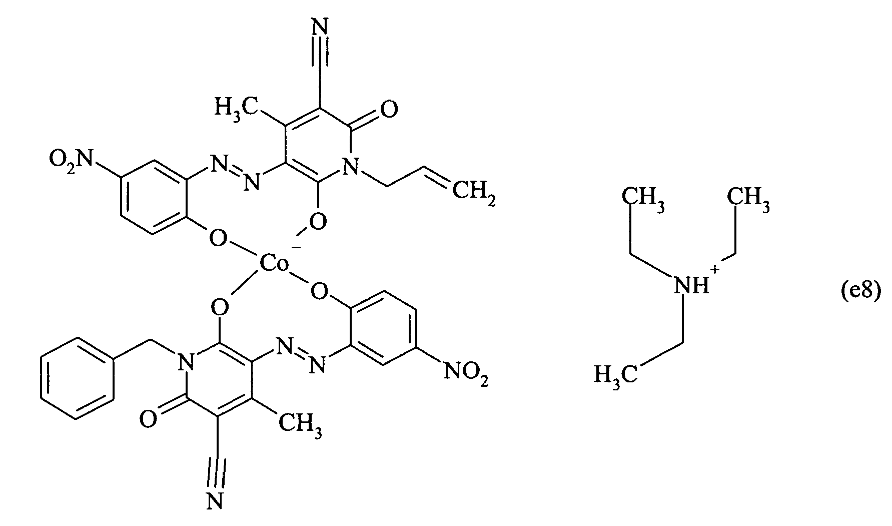

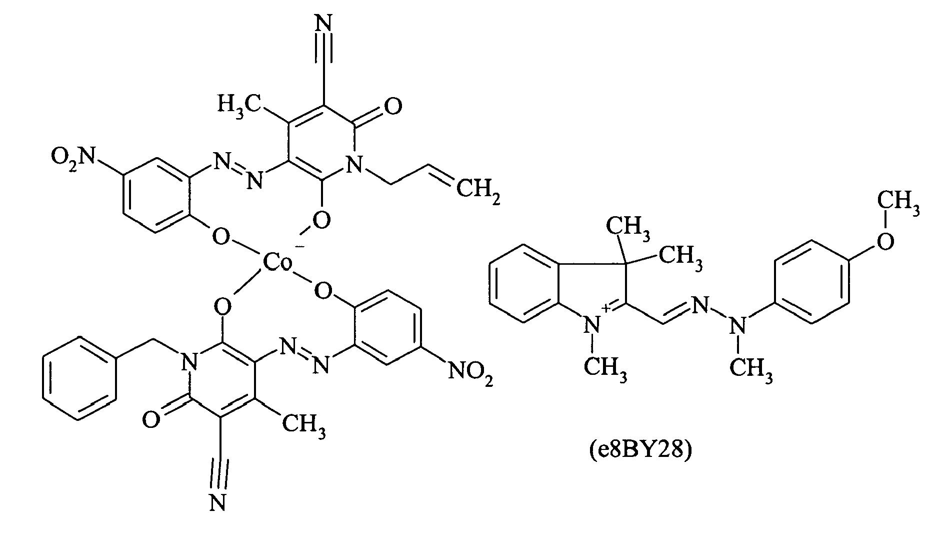

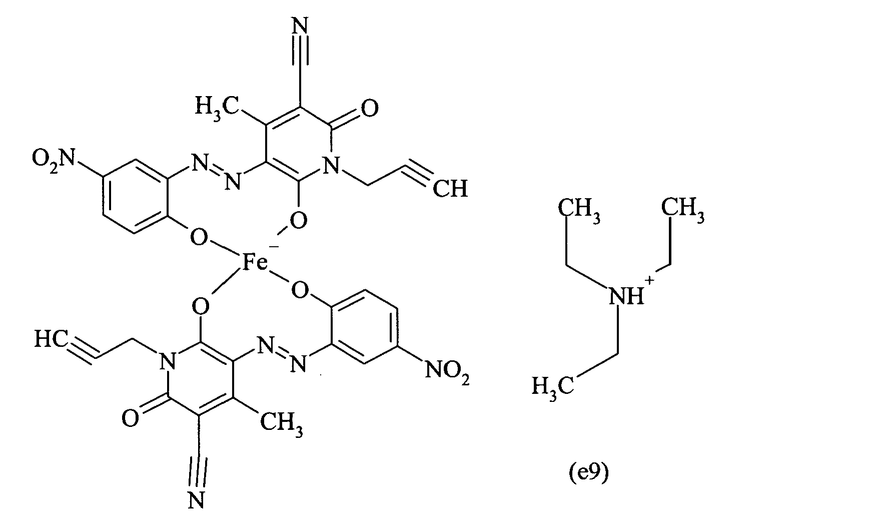

### Preparation of the compounds of formula (I) with A* and A** each being H

In the following, the compounds of formula (I) with A* and A** in formula (I) being H and the compounds of formula (III), 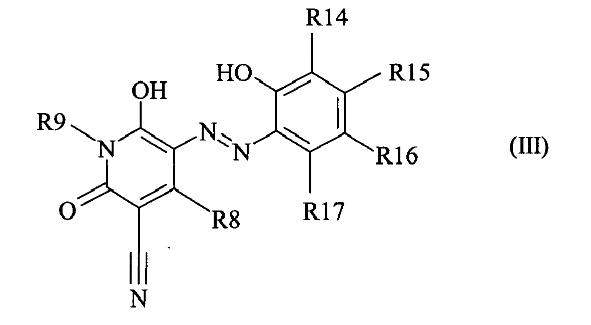 with R8, R9, R14, R15, R16 and R17 being as defined in formula (II), also in all their preferred embodiments as described above, are called azo ligands.

In the case that R9 represents C₁₋₄ alkyl or-NH-phenyl, the compounds of formula (III) are known compounds, e.g. from the WO 2006/106110 A, and can be prepared according to or in analogy to known procedures.

The compounds of formula (I) with A* and A** each being H, especially the compounds of formula (d1), (d2), (d3), (d4) and (d5), are preferably prepared by an azo coupling reaction of the respective compound of formula (Ia), also called coupling agent, with the respective compound of formula (Ib), also called diazo component; the compound of formula (Ib) being preferably prepared by diazotization reaction of the respective compound of formula (Ic), also called amine compound; 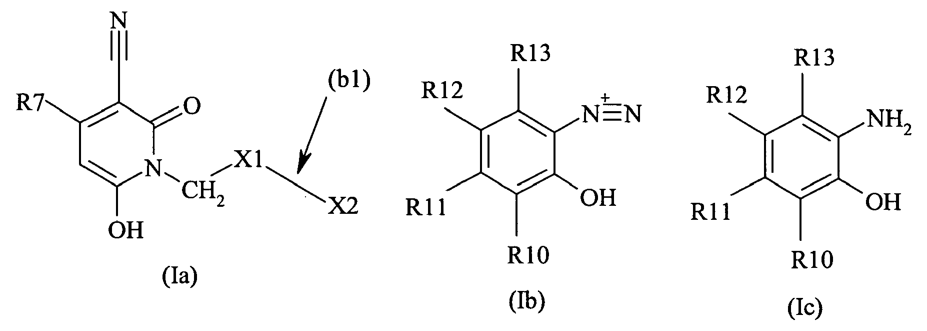 wherein (b1), R7, R10, R11, R12, R13, X1 and X2 have the same meaning as described above, also with all their preferred embodiments.

The diazo component has preferably chloride Cl- as counter ion, since the diazotization reaction of the amine compound preferably is done in aqueous hydrochloride acid.

The amine compounds are known substances and can be prepared according to or in analogy to known procedures.

It is possible to use more than one amine component and/or more than one coupling agent resulting in the respective mixture of the azo ligands.

The azo coupling reaction is carried out in water, non-aqueous solvents and in mixtures thereof. Non-aqueous solvents are preferably selected from the group consisting of alcohols, more preferably methanol, ethanol, propanol, butanol, pentanol, dipolar aprotic solvents, preferably dimethylformamide (DMF), DMSO, dimethylacetamide or N-methyl-pyrrolidinone (NMP) and pyridine, and water-immiscible solvents, preferably toluene or chlorobenzene. More preferably the azo coupling reaction is carried out in water.

The azo coupling reaction is preferably carried out with a stoichiometric ratio of coupling component and diazo component. The azo coupling reaction is generally done at a temperature of from -30°C to 100°C, preference being given to temperatures of-10°C to 30°C, and particular preference to temperatures of-5°C to 20°C.

The azo coupling reaction may be carried out in an acidic as well as an alkaline medium. Preference is given to pH < 10, particular preference to pH 3 to 9.

Preferably the azo ligand is isolated following standard methods, in case of a precipitate preferably by filtration followed preferably by washing and drying.

A further subject of the invention is a compound of formula (Ia), also in all its preferred aspects as described above.

The compound of formula (Ia), i.e. the coupling agent, is preferably prepared by a condensation reaction of a respective amine compound with cyanoacetic acid ethylester resulting in an intermediate amide, and successive condensation reaction of the intermediate amide with acetyl acetic acid ethylester under basic conditions. The amide can be isolated after precipitation or by distillation, preferably it is not isolated and the two steps are carried out without interruption.

The condensation reaction is carried out in non-aqueous solvents and in mixtures thereof. Non-aqueous solvents are preferably selected from the group consisting of alcohols, more preferably methanol, ethanol, propanol, butanol, pentanol, further dipolar aprotic solvents, preferably dimethylformamide (DMF), DMSO, dimethylacetamide or N-methyl-pyrrolidinone (NMP) and pyridine, and further water-immiscible solvents, preferably toluene, chlorobenzene, hexane, cyclohexane or heptane. More preferably the condensation reaction is carried out in toluene or ethanol.

The condensation reaction is preferably carried out with a stoichiometric ratio of amine, cyanoacetic acid ethylester and acetylacetic acid ethylester.

The condensation reaction is preferably done at a temperature of from 0°C to 200°C, more preferably of from 10°C to 180°C, even more preferably of from 25°C to 150°C.

Preferably, water formed during reaction is distilled off during the reaction.

The reaction time is preferably of from 30 min to 30 hours, more preferably of from 1 hour to 24 hours.

The condensation reaction is preferably carried out in the presence of a organic or inorganic base as catalyst, preferably selected from the group consisting of alkaline hydroxides, preferably NaOH and KOH, further organic aromatic amines, preferably pyridine, further organic alkylamines, preferably triethylamine, piperidine and lutidine, further sodium- or potassium alcoholates, preferably sodium methoxide or sodium ethoxide, and further basic ion exchange resins.

Preferably the coupling agent is isolated following standard methods, in case of a precipitate preferably by filtration followed preferably by washing and drying, in case of a solution, the solution is preferably concentrated until precipitation, preferably by distillation.

### Preparation of the compounds of formula (I) with the residues A* and A** together representing a group of formula (II)

A further subject of the invention is a process for the preparation of compounds of formula (I) with the residues A* and A** together representing a group of formula (II), as well as of compounds of formula (I) with the residues A* and A** together representing a group of formula (II) in all the preferred aspects of the formula (I) as described above, especially of the compounds of formulae (e1), (e1BY28), (e1a1), (e1a2), (e1a3), (e1a4), (e1a5), (e1a6), (e1a7), (e2), (e2BY28), (e2b2), (e3), (e3BY28), (e4), (e4BY28), (e4a2), (e5), (e5BY28), (e6), (e6BY28), (e6a2), (e7), (e7BY28), (e8) or (e8BY28),
by a complexing reaction of a compound of formula (I) with A* and A** being H and a compound of formula (III) with a metal salt, in the presence of Cat⁺ or with in situ formation of Cat+; with the compound of formula (I) with A* and A** being H and the compound of formula (III) preferably being prepared by an azo coupling reaction of the respective diazo components and the respective coupling agents;
or by metathesis of respective precursor salts.

Particularly the compounds of formulae (e1), (e2), (e3), (e4), (e5), (e6), (e7) or (e8) are prepared by a complexing reaction of a respective metal salt with the respective compounds of formulae (d1), (d2), (d3), (d4) or (d5).

Preferably, the complexing reaction is done with a solution of one equivalent of a metal salt and with a boiling solution of two equivalents of the respective compound of formula (IV).

Preferably, the complexing reaction is done with a trivalent metal salt. In another preferred embodiment of the invention, particularly in case of the compounds of formulae (e1), (e2), (e3), (e4), (e5), (e6), (e7) or (e8), the metal of the metal salt is derived from a divalent metal, and the complexing reaction is carried out in the presence of preferably 2.5 to 4, more preferably 2.9 to 3.2, especially 3 equivalents of trialkylamine for each equivalent of ligand under aerobic conditions. This ensures, that the divalent metal atom is converted during the complexing reaction to a trivalent metal atom, and that the metal atom is incorporated into its four-fold coordination in the complex, resulting in an anionic charge on the final complex.

It is possible to use more than one, preferably 1, 2 or 3, more preferably 1 or 2, metal salts. It is possible also to use more than one, preferably 1, 2, 3, 4, 5 or 6, more preferably of 1, 2, 3 or 4, even more preferably of 1 or 2, compounds of formula (I) with A* and A** each being H and/or compounds of formula (III), i.e. to use more than one azo ligand, and a combination of these measures is also possible, resulting in homo- and/or heteroleptic complexes. Preferably, only one metal salt and only one or two azo ligands are used, resulting in homoleptic or heteroleptic complexes.
Preferably, the total amount of metal salt is used in the required stoichiometric amount with regard to the total amount of azo ligand, i.e. the ratio is preferably one equivalent of metal salt to two equivalents of azo ligand.

The azo ligand can be added to the metal salt or vice versa.

In one preferred embodiment of the invention Cat⁺ is present during the complexing reaction, in another preferred embodiment of the invention Cat⁺ is formed during the complexing reaction, more preferably Cat⁺ is formed during the complexing reaction when a metal salt derived from a divalent metal is used in the presence of triethylamine under aerobic conditions in the complexing reaction, with the metal salt being especially preferably CoSO₄*7H₂O or FeSO₄*7H₂O.

In another preferred embodiment of the invention the compound of formula (I) with the residues A* and A** together representing a group of formula (II), is prepared by metathesis, preferably by mixing the respective precursor salts.

The complexing reaction and the metathesis can be carried out in suspension or in solution, preferably in suspension. The metal salt is being used preferably in form of a solution. Preferably the azo ligand is present as a solution in the complexing reaction.

The solvent preferably used in the complexing reaction and the metathesis is water, a non-aqueous solvent or a mixture thereof. The non-aqueous solvent is preferably selected from the group consisting of C₁₋₈ alcohols, nitriles, preferably acetonitrile, ketones, preferably acetone, aromatic solvents, preferably toluene or chlorobenzene, and dipolar aprotic solvents, preferably DMF, DMSO, NMP, pyridine and mixtures thereof. More preferred solvents are C₁₋₈ alcohols, especially ethanol, acetonitrile and pyridine.

It is also possible to add the metal salt already at an earlier stage of the synthesis of the azo ligand or their precursors, preferably before, during or after the azo coupling reaction, more preferably after the azo coupling reaction to the resulting suspension or solution of the azo ligand.

Even more preferably the azo ligand is isolated and dried after synthesis, and the complexing reaction is carried out in a separate step.

Preferably the ligands are present as a suspension in the complexing reaction or during metathesis.

The complexing reaction and the metathesis are preferably done at a temperature of from 0°C to 200°C, more preferably of from 5°C to 170°C, even more preferably of from 20°C to 150°C, especially the complexing reaction is carried out under reflux at the reflux temperature of the solvent system used and at atmospheric pressure.

Preferably the compound of formula (I) with the residues A* and A** together representing a group of formula (II) is isolated following standard methods, usually the compound of formula (I) with the residues A* and A** together representing a group of formula (II) forms a precipitate which is isolated, preferably by filtration, and preferably followed by drying.

The metal salt is a derived from a divalent or a trivalent metal with the metal selected preferably from the group consisting of Co, Al, Fe and Cr. The salts of these metals are preferably sulfates, halides (preferably fluoride, chloride, bromide, iodide, more preferably chloride and bromide, especially chlorides) and salts of organic acids, preferably acetates, and their respective hydrates.

In case of a divalent metal the metal has to be converted to its trivalent form. Preferably this is done during complexing reaction at presence of triethylamine under aerobic conditions.

Preferred metal salts are derived from Co, Fe and Al. More preferred metal salts are for example cobalt-, iron- or aluminium-halides, more preferable chlorides, cobalt-, iron- or aluminium-sulfates; cobalt- or aluminium-acetates, and their respective hydrates, especially preferably AlCl₃, Al₂(SO₄)₃, Al₂(SO₄)₃*18 H₂O, CoSO₄*7 H₂O and FeSO₄* 7H₂O, FeCl₃*H₂O, FeCl₃*6H₂O, Fe(acetylacetate)₃, Fe₂(SO₄)₃*xH₂O or iron salts of organic acids, preferable formate, gluconate, citrate and oxalate.

More preferably the complexing reaction is done with a metal salt derived from a divalent metal under aerobic conditions, with the metal salt preferably being CoSO₄*7H₂O or FeSO₄*7H₂O, in the presence of triethylamine.

A further subject of the invention is the use of a compound of formula (Ia) for the preparation of a compound of formula (I), preferably of a compound of formula (I) with A* and A** each being H; preferably the use of a compound of formula (Ia) as a coupling agent for the preparation of a compound of formula (I) by an azo coupling reaction.

A further subject of the invention is the use of a compound of formula (I) with A* and A** each being H, also in all its preferred embodiments, especially of a compound of formula (d1), (d2), (d3), (d4) or (d5) as a ligand, preferably as a ligand in azo metal complex dyes.

A further subject of the invention is the use of a compound of formula (I), the use of a compound of formula (I) in all the preferred aspects of the formula (I) as described above, and more preferably the use of a compound of formula (d1), (d2), (d3), (d4), (d5), (e1), (e1BY28), (e1a1), (e1a2), (e1a3), (e1a4), (e1a5), (e1a6), (e1a7), (e2), (e2BY28), (e2b2), (e3), (e3BY28), (e4), (e4BY28), (e4a2), (e5), (e5BY28), (e5a2), (e5a4), (e6), (e6BY28), (e6a2), (e7), (e7BY28), (e8), (e8BY28) or (e9) in an optical layer, preferably in an optical layer for optical data recording.

A further subject of the invention is the use of a compound of formula (I), the use of a compound of formula (I) in all the preferred aspects of the formula (I) as described above, and more preferably the use of a compound of formula (d1), (d2), (d3), (d4), (d5), (e1), (e1BY28), (e1a1), (e1a2), (e1a3), (e1a4), (e1a5), (e1a6), (e1a7), (e2), (e2BY28), (e2b2), (e3), (e3BY28), (e4), (e4BY28), (e4a2), (e5), (e5BY28), (e5a2), (e5a4), (e6), (e6BY28), (e6a2), (e7), (e7BY28), (e8), (e8BY28) or (e9), as a dye in an optical layer, preferably in an optical layer for optical data recording.

A further subject of the invention is an optical layer comprising at least one compound of formula (I), with the compound of formula (I) also in all its described embodiments, particularly at least one compound of formula (d1), (d2), (d3), (d4), (d5), (e1), (e1BY28), (e1a1), (e1a2), (e1a3), (e1a4), (e1a5), (e1a6), (e1a7), (e2), (e2BY28), (e2b2), (e3), (e3BY28), (e4), (e4BY28), (e4a2), (e5), (e5BY28), (e5a2), (e5a4), (e6), (e6BY28), (e6a2), (e7), (e7BY28), (e8), (e8BY28) or (e9); and the use of said optical layer for optical data recording media. An optical layer according to the invention may also comprise a mixture of two or more, preferably of two or three, more preferably of two compounds of formula (I). A further subject of the invention therefore is an optical data recording medium comprising an optical layer comprising at least one compound of formula (I).

Further, the invention relates to a method for producing an optical layer comprising the following steps
(a) providing a substrate,
(b) dissolving at least one compound of formula (I), particularly at least one compound of formula (d1), (d2), (d3), (d4), (d5), (e1), (e1BY28), (e1a1), (e1a2), (e1a3), (e1a4), (e1a5), (e1a6), (e1a7), (e2), (e2BY28), (e2b2), (e3), (e3BY28), (e4), (e4BY28), (e4a2), (e5), (e5BY28), (e5a2), (e5a4), (e6), (e6BY28), (e6a2), (e7), (e7BY28), (e8), (e8BY28) or (e9), in an organic solvent to form a solution,
(c) coating the solution (b) on the substrate (a),
(d) evaporating the solvent to form an optical layer.

### (a) Substrate

The substrate, which functions as support for the layers applied thereto, is advantageously semi-transparent (transmittance T>10%) or preferably transparent (transmittance T>90%). The support can have a thickness of from 0.01 to 10 mm, preferably from 0.1 to 5 mm.

Suitable substrates are, for example, glass, minerals, ceramics and thermosetting or thermoplastic plastics. Preferred supports are glass and homo- or co-polymeric plastics. Suitable plastics are, for example, thermoplastic polycarbonates, polyamides, polyesters, polyacrylates and polymethacrylates, polyurethanes, polyolefins, polyvinyl chloride, polyvinylidene fluoride, polyimides, thermosetting polyesters and epoxy resins. The most preferred substrates are polycarbonate (PC) or polymethylmethacrylate (PMMA).

The substrate can be in pure form or may also comprise customary additives, for example UV absorbers as light-stabilizers for the optical layer.

The substrate is advantageously transparent over at least a portion of the range from 350 to 500 nm, so that it is permeable to at least 90% of the incident light of the writing or readout wavelength.

### (b) Organic solvents

Organic solvents are selected from C₁₋₈ alcohols, halogen substituted C₁₋₈ alcohols, C₁₋₈ ketones, C₁₋₈ ethers, halogen substituted C₁₋₄ alkanes, nitriles, preferably acetonitrile, or amides, or mixtures thereof.

Preferred C₁₋₈ alcohols or halogen substituted C₁₋₈ alcohols are for example methanol, ethanol, isopropanol, diacetone alcohol (DAA), 2,2,3,3-tetrafluoropropan-1-ol, trichloroethanol, 2-chloroethanol, octafluoropentanol or hexafluorobutanol, more preferred 2,2,3,3-tetrafluoropropan-1-ol.

Preferred C₁₋₈ ketones are for example acetone, methylisobutylketone, methylethylketone, or 3-hydroxy-3-methyl-2-butanone.

Preferred halogen substituted C₁₋₄ alkanes are for example chloroform, dichloromethane or 1-chlorobutane.

Preferred amides are for example DMF, dimethylacetamide or NMP.

### (c) Coating methods

Suitable coating methods are, for example, immersion, pouring, brush-coating, blade-application and spin-coating, as well as vapor-deposition methods carried out under a high vacuum. When pouring methods are used, solutions in organic solvents are generally used. When solvents are employed, care should be taken that the supports used are insensitive to those solvents. The optical layer is preferably applied by spin-coating with a dye solution.

### (d) Optical layer

The optical layer is preferably arranged between the transparent substrate and the reflecting layer. The thickness of the recording layer is from 10 to 1000 nm, preferably from 30 to 300 nm, more preferably from 70 to 250 nm, especially about 80 nm, for example from 60 to 120 nm.

The optical layer comprises a compound of formula (I), preferably in an amount sufficient to have a substantial influence on the refractive index, more preferably at least 30 % by weight, even more preferably at least 60 % by weight, especially at least 80 % by weight, the % by weight always based on the total weight of the optical layer.

Further customary components are stabilizers, for example ¹0₂-, triplet- or luminescence quenchers, melting-point reducers, decomposition accelerators or any other additives that have already been described in optical data recording media. Preferably, stabilizers or fluorescence-quenchers are added if desired.

Stabilizers, ¹0₂-, triplet- or luminescence-quenchers are, for example, metal complexes ofN- or S-containing enolates, phenolates, bisphenolates, thiolates or bisthiolates, hindered phenols and derivatives thereof such as o-hydroxyphenyl-triazoles or -triazines or other UV absorbers, such as hindered amines (TEMPO or HALS, as well as nitroxides or NOR-HALS), and also as cations diimmonium, Paraquat™ or Orthoquat salts, such as ®Kayasorb IRG 022, ®Kayasorb IRG 040, optionally also as radical ions, such as N,N,N',N'-tetrakis(4-dibutylaminophenyl)-p-phenylene amine-ammonium hexafluorophosphate, hexafluoroantimonate or perchlorate. The latter are available from Organica (Wolfen/DE); ®Kayasorb brands are available from Nippon Kayaku Co. Ltd. In a preferred aspect, the present invention provides for an optical layer suitable for high-density recording material, e.g. of the WORM disc format, in a laser wavelength range of from 350-450nm, preferably around 405 nm.

### Preparation of the optical data recording medium

A method for producing an optical data recording medium comprising an optical layer according to the invention usually comprises the following additional steps
(e) applying a metal layer, also called reflective layer, onto the optical layer,
(f) applying a second polymer based layer to complete the disk, also called cover layer or protective layer.

### (e) Reflective layer

The application of the metallic reflective layer is preferably effected by sputtering, vapor-deposition in vacuum or by chemical vapor deposition (CVD). The sputtering technique is especially preferred for the application of the metallic reflective layer.

Reflecting materials suitable for the reflective layer include especially metals, which provide good reflection of the laser radiation, used for recording and playback, for example the metals of Main Groups III, IV and V and of the Sub-groups of the Periodic Table of the Elements. Al, In, Sn, Pb, Sb, Bi, Cu, Ag, Au, Zn, Cd, Hg, Sc, Y, La, Ti, Zr, Hf, V, Nb, Ta, Cr, Mo, W, Fe, Co, Ni, Ru, Rh, Pd, Os, Ir, Pt, Ce, Pr, Nd, Pm, Sm, Eu, Gd, Tb, Dy, Ho, Er, Tm, Yb and Lu and alloys thereof are especially suitable. Special preference is given to a reflective layer of aluminum, silver, copper, gold or an alloy thereof, on account of their high reflectivity and ease of production.

### (f) Cover layer

Materials suitable for the cover layer include plastics, which are applied in a thin layer to the support or the uppermost layer either directly or with the aid of adhesive layers. The material of the cover layer may for example be the same as the material of the substrate. It is advantageous to select mechanically and thermally stable plastics having good surface properties, which may be modified further.

The plastics may be thermosetting plastics and thermoplastic plastics. Preference is given to radiation-cured (e.g. using UV radiation) protective layers, which are particularly simple and economical to produce. A wide variety of radiation-curable materials are known. Examples of radiation-curable monomers and oligomers are acrylates and methacrylates of diols, triols and tetrols, polyimides of aromatic tetracarboxylic acids and aromatic diamines having C₁-C₄alkyl groups in at least two ortho-positions of the amino groups, and oligomers with dialkylmaleinimidyl groups, e.g. dimethyl maleinimidyl groups.

A high-density optical data recording medium according to the invention therefore preferably is a recordable optical disc comprising: a first substrate, which is a transparent substrate with grooves, a optical layer (recording layer), which is formed on the first substrate surface using the compound of formula (I), a reflective layer formed on the optical layer, a second substrate, which is a transparent substrate connected to the reflective layer with an attachment layer.

The optical data recording medium according to the invention is preferably a recordable optical disc of the WORM type. It may be used, for example, as a playable HD-DVD (high density digital versatile disc) or Blu-ray® disc, as storage medium for a computer or as an identification and security card or for the production of diffractive optical elements, for example holograms.

The optical data recording media according to the invention may also have additional layers, for example interference layers. It is also possible to construct optical data recording media having a plurality of (for example two) recording layers. The structure and the use of such materials are known to the person skilled in the art. Preferred, if present, are interference layers that are arranged between the recording layer and the reflecting layer and/or between the recording layer and the substrate and consist of a dielectric material of Ti0₂, Si₃N₄, ZnS or silicone resins.

These optical data recording media according to the invention can be produced by processes known in the art.

### Readout methods

The structure of the optical data recording medium according to the invention is governed primarily by the readout method; known function principles include the measurement of the change in the transmission or, preferably, in the reflection, but it is also known to measure, for example, the fluorescence instead of the transmission or reflection.

When the optical data recording medium is structured for a change in reflection, the following structures can be used: transparent support / recording layer (optionally multilayered) / reflective layer and, if expedient, protective layer (not necessarily transparent); or support (not necessarily transparent) / reflective layer / recording layer and, if expedient, transparent protective layer. In the first case, the light is incident from the support side, whereas in the latter case the radiation is incident from the recording layer side or, where applicable, from the protective layer side. In both cases the light detector is located on the same side as the light source. The first-mentioned structure of the recording material to be used according to the invention is generally preferred.

When the optical data recording medium is structured for a change in light transmission, the following different structure comes into consideration: transparent support/recording layer (optionally multilayered) and, if expedient, transparent protective layer. The light for recording and for readout can be incident either from the support side or from the recording layer side or, where applicable, from the protective layer side, the light detector in this case always being located on the opposite side.

Suitable lasers are those having a wavelength of 330-500 nm, for example commercially available lasers having a wavelength of 405 to 414 nm, especially semi-conductor lasers. The recording is done, for example, point for point, by modulating the laser in accordance with the mark lengths and focusing its radiation onto the recording layer. It is known from the specialist literature that other methods are currently being developed which may also be suitable for use.

The process according to the invention allows the storage of information with great reliability and stability, distinguished by very good mechanical and thermal stability and by high light stability and by sharp boundary zones of the pits. Special advantages include the high contrast, the low jitter and the surprisingly high signal/noise ratio, so that excellent readout is achieved.

The readout of information is carried out according to methods known in the art by registering the change in absorption or reflection using laser radiation.

The invention accordingly relates also to a method for the optical data recording, storage and playback of information, wherein an optical data recording medium according to the invention is used. The recording and the playback advantageously take place in a wavelength range of from 330 to 500 nm.

The compounds of formula (I) provide for particularly preferable properties when used in optical layers for optical data recording media according to the invention. They possess the required optical characteristics, demonstrated when used in the form of a solid film:
- an advantageously homogeneous, amorphous and low-scattering optical layer,
- a high refractive index at the longer wavelength flank of the absorption band, which preferably achieves n values of the refractive index of from 1.0 to 3.0 in the range of from 330 to 500 nm,
- a high sensitivity under laser radiation of high power density and good playback characteristics in the desired spectral range,
- an enhanced photosensitivity and stability (in daylight and under laser radiation of low power density) compared to dyes already known in the art,
- an uniform script width and a high contrast,
- an absorption maximum (λ max) in the preferred range between 330 nm and 500 nm as being preferred for blue laser applications, more precisely from 380 to 460 nm,
- a decomposition point (DP) in the preferred temperature range between 180°C and 300°C, more precisely 200°C to 290°C
- a sufficient heat release (HR)

Recording performance of a compound is related to specific parameters measured on disc like:
- a low simulated bit error rate (SbER)
- a low inner parity error rate (PI error)
- a high reflectivity (R)
- a low laser recording power (Pw: power, or OPC: optimum power control): the lower the better
- good readout stability at different laser reading powers
- an appropriate partial response signal to noise ratio (PRSNR): the higher the better

The absorption edge is surprisingly steep even in the solid phase.

The compounds of formula (I) also show a narrow decomposition temperature of 180 to 350°C, fitting with the thermal requirements. Additionally, these compounds show a high solubility in organic solvents, which is ideal for the spin-coating process to manufacture optical layers.

The use of a compound of formula (I) in an optical layer for optical data recording allows unexpectedly data recoding at higher speeds than the conventional 1X speed in HD-DVD and Blu-ray-discs.

As a result of the use of the dyes of the invention, the recording media of the invention advantageously have homogeneous, amorphous and low scattering recording layers. Further advantages is the light stability in day light and under laser radiation of 0.4 mW, combined with a high sensitivity under laser radiation of moderate, this means as low as possible, power density (OPC preferably less than 8.0 mW for 1X speed and preferably less than 11 mW for 2X speed), the good thermal and storage stability. Especially in case of recording at higher speed, the OPC required should be as low as possible.

### Examples

### UV-vis

For UV-vis spectra, λ max and ε values of a compound are determined by using an UV-vis spectrophotometer, the compound was dissolved in CH₂Cl₂, DMSO or in tfp. The values are obtained by balancing the measurements performed on compound solutions at three different concentrations.

### Melting point (MP)

For the determination of melting point, the compound or the composition is incorporated in a glass capillary. The capillary was heated using the following profile: temperature range from 20 to 350 °C, heating rate 2 °C/min.

### Thermal Decomposition: Decomposition point (DP) and heat release (HR)

For the determination of DP and HR, the compound is incorporated into a sealed aluminum pan. Analysis conditions are as following: Temperature range from 25 to 400°C, heating rate 10°C/min, nitrogen flow of 50 ml/min. Values are determined by single measurement. Additionally, thermal decomposition is also being observed while measuring the melting point.

### Partial response signal to noise ratio (PRSNR)

A definition and the measuring techniques of PRSNR are described in a book available from DVD Format Logo Licensing Co., Ltd. for example, Annex H of Version 0.9, PART 1 Physical Specifications, DVD Specifications for High Density Read-Only Disk. The higher the PRSNR the better.

### Simulated bit error rate (SbER)

A definition and the measuring techniques of SbER are described in a book available from DVD Format Logo Licensing Co., Ltd. for example, Annex H of Version 0.9, PART 1 Physical Specifications, DVD Specifications for High Density Read-Only Disk. The lower the SbER the better.

PRSNR and SbER are measured in a state in which information has been recorded in the adjacent tracks.

### Reflectivity (R)

A definition and the measuring techniques for the light reflectivity (R) is described in a book available from DVD Format Logo Licensing Co., Ltd. for example, Annex D of Version 0.9, PART 1 Physical Specifications, DVD Specifications for High Density Read-Only Disk. The higher the R the better.

### Cycle number

The degree of degradation of various parameters, e.g. of the PRSNR and SbER, due to repetitive read out is measured. The higher the cycle number until reaching the minimum specifications or a comparable performance the better.

"Ex." means example, "Comp. Ex." means comparative example.

### Example 1

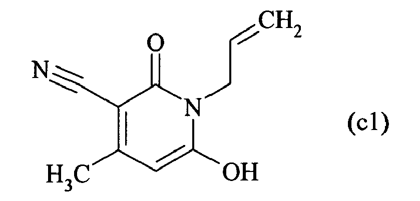

34.65 g of ethyl cyanoacetate were added dropwise to 17.48 g of allylamine. The resulting mixture was refluxed for 1hour. Temperature was cooled down to 70°C and 43.38 g of ethyl acetoacetate were added dropwise followed by 48 g of piperidine and 166 ml of toluene respectively. The resulting mixture was refluxed overnight. Toluene was distilled off, 160 ml of water were added and the mixture was allowed to cool to room temperature.

The resulting slurry was transferred dropwise to 150 ml of cold methanol (ice bath). During the transfer, the pH is maintained to pH 1 by addition of concentrated aqueous HCl. The resulting yellowish precipitate was filtered, washed 5 times with each 200 ml of water and dried under vaccum at 60°C for 24 hours. 37.7 g of compound of formula (c1) were obtained as a white solid.

### Examples 2 and 3

The preparation according to example 1 was done using cyanoacetate and acetoacetate in a stoichiometric ratio with regard to the respective amine compound to yield the compounds of formula (c2) and (c3). The combinations and details are given in table (A1).

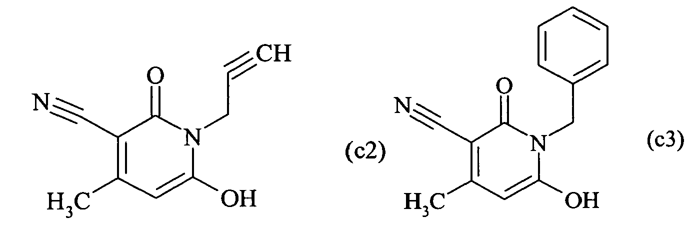

| **Table (A1)** | | | | | | |
|---|---|---|---|---|---|---|
| **Ex.** | **cyanoacetate** | **acetoacetate** | **amine compound** | | **compound of formula** | |
| | **[g]** | **[g]** | | **[g]** | | **[g]** |
| **1** | 34.7 | 43.4 | Allylamine | 17.5 | (c1) | 37.7 |
| **2** | #### | #### | Propargylamine | ### | (c2) | ### |
| **3** | 57.8 | 73 | Benzylamine | 54.7 | (c3) | 93.6 |

Table A2 shows the phys-chem properties of the compounds of formulae (c1) to (c3).

| **Table (A2)** | | | | |
|---|---|---|---|---|
| **Ex.** | **Compound of formula** | **λ max [nm]** | **ε (at λ max) [L / g * cm]** | **MP/ DP [°C]** |
| **1** | (c1) | 231 | 32.7 | 227 (MP) |
| **2** | (c2) | 332 | 76.3 | 187 (DP) |
| **3** | (c3) | 230 | 35.2 | 245 (MP) |

### Example 4

8.68 g of 4-nitro-2-aminophenol were added to 100 ml of water followed by dropwise addition of 16.1 g of concentrated aqueous HCl. Temperature was cooled to 0°C and 10.4 ml of a aqueous solution of sodium nitrite (33.3% by weight) were added dropwise, keeping the temperature below 5°C. The yellow mixture was stirred at this temperature for 1hour. The mixture was then pumped in a mixture containing 9.51 g of compound of formula (c1) and 12.4 g of sodium acetate in 200 ml of water. After complete addition, the resulting mixture was stirred 1hour at room temperature. The resulting orange precipitate was filtered, washed 6 times of each 250 ml of water and dried under vacuum at 60°C for 24 hours. 16.12 g of compound of formula (d1) were obtained as a yellow solid.

### Examples 5 to 8

The preparation according to example 4 was done using the respective coupling agent in a stoichiometric ratio with regard to the respective amine compound to yield the compounds of formula (d2) to (d5). The combinations and details are given in table (A3). The amine compound for diazotization in case of examples 4 to 6 was 2-amino-4-nitro-phenol (NAP), the amine compound for diazotization in case of examples 7 and 8 was 2-amino-4-nitro-6-acetamidophenol (ANAP).

| **Table (A3)** | | | | | |
|---|---|---|---|---|---|
| **Ex.** | **coupling agent** | | **NAP/ANAP** | **compound of formula** | |
| | **compound of formula** | **[g]** | **[g]** | | **[g]** |
| **4** | (c1) | 9.5 | 8.7/- | (d1) | 16.1 |
| **5** | (c2) | 21.9 | 21.5/- | (d2) | 36.0 |
| **6** | (c3) | 30.0 | 21.7/- | (d3) | 44.0 |
| **7** | (c1) | 13.5 | -/15 | (d4) | 22.2 |
| **8** | (c2) | 12.4 | -/13.9 | (d5) | 15.1 |

Table A4 shows the phys-chem properties of the compounds of formulae (d1) to (d5).

| **Table (A4)** | | | | |
|---|---|---|---|---|
| **Ex.** | **Compound of formula** | **λ max [nm]** | **ε (at λ max) [L / g * cm]** | **MP/ DP [°C]** |
| **4** | (d1) | 439 | 99 | 272 (DP) |
| **5** | (d2) | 439 | 69 | 238 (DP) |
| **6** | (d3) | 441 | 75 | 310 (DP) |
| **7** | (d4) | ### | ### | ### |
| **8** | (d5) | 432 | 57 | ### |

### Example 9

7.1 g of compound of formula (d1), 2.81 g of CoSO₄·7H₂O and 100 ml of acetonitrile were refluxed for 20 minutes. 6.13 g of triethylamine were added dropwise and the resulting mixture was refluxed under aerobic conditions for 1h 30 min. After being cooled to room temperature, the solution was filtered and the filtrate was distilled. To the resulting purple slurry were added dropwise 100 ml of ethanol and the mixture was refluxed for 1 hour. After being cooled to room temperature, the greenish-brown precipitate was filtered, washed with 50 ml of ethanol, 300 ml of water and dried under vaccum at 60°C for 24 hours. 8.06 g of compound of formula (e1) were obtained as a greenish-brown solid.

### Example 10 to 13

The preparation according to example 9 was done using 2 equivalents of the respective azo ligand with regard to one equivalent of CoSO₄·7H₂O to yield the compounds of formula (e2) to (e5). The combinations and details are given in table (A5).

### Example 14

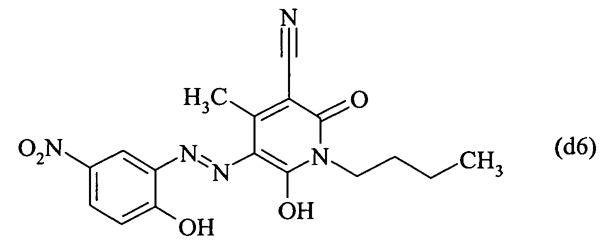

The compound of formula (e6) was prepared according to the example 9 with the sole difference, that instead of 7.1 g of compound of formula (d1), 9.9 g of compound (d1) and 10.4 g of compound of formula (d6) were used.

### Example 15 and 16

The preparation according to example 14 was done using the respective two different azo ligands with regard to one equivalent of CoSO₄·7H₂O to yield the compounds of formula (e7) to (e8). The combinations and details are given in table (A5).

| **Table (A5)** | | | | | |
|---|---|---|---|---|---|
| **Ex.** | **Azo ligand** | | **CoSO₄·7H₂O** | **compound of formula** | |
| | **compound of formula** | **[g]** | **[g]** | | **[g]** |
| **9** | (d1) | 7.1 | 2.8 | (e1) | 8.1 |
| **10** | (d2) | 17.1 | 6.1 | (e2) | 15.3 |
| **11** | (d3) | 12.6 | 4.4 | (e3) | 13.6 |
| **12** | (d4) | 10.0 | 3.4 | (e4) | 8.3 |
| **13** | (d5) | 15.0 | 5.1 | (e5) | 15.2 |
| **14** | (d1) (d6) | 9.9 10.4 | 7.9 | (e6) | 21.4 |
| **15** | (d3) (d6) | 10.1 9.3 | 7.0 | (e7) | 17.5 |
| **16** | (d1) (d3) | 8.9 10.1 | 7.0 | (e8) | 16.6 |

Table A6 shows the phys-chem properties of the compounds of formulae (e1) to (e8).

| **Table (A6)** | | | | |
|---|---|---|---|---|
| **Ex.** | **Compound of formula** | **λ max [nm]** | **ε (at λ max) [L / g * cm]** | **MP/ DP [°C]** |
| **9** | (e1) | 482 | 58 | 309 (DP) |
| **10** | (e2) | 514 | 55 | 315 (DP) |
| **11** | (e3) | 482 | 51 | 316 (DP) |
| **12** | (e4) | 486 | 57 | 232 (DP) |
| **13** | (e5) | ### | ### | ### (DP) |
| **14** | (e6) | ### | ### | ### (DP) |
| **15** | (e7) | ### | ### | 303 (DP) |
| **16** | (e8) | 482 | 51 | 316 (DP) |

### Example 17

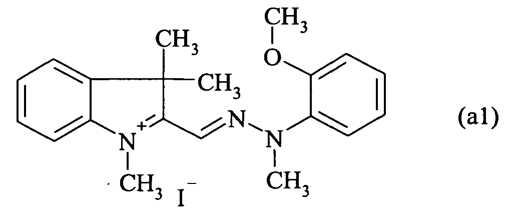

32.2 g of conc. aqueous HCl were added dropwise to a solution composed of 12.4 g of 2-methoxyaniline in 100 ml of water. Temperature was decreased to 0°C with an ice bath and 20.8 ml of a solution of aqueous sodium nitrite (33.3% by weight) were added dropwise while temperature was maintained below 5°C. The resulting solution was stirred at 0°C for 1hour and added dropwise to a mixture composed of 17.6 g of 1,3,3-trimethyl-2-methyl-indoline, 31.8 g of Na₂CO₃, 100 ml of methanol and 30 ml of water at 10°C.

After complete addition, the resulting mixture was stirred for 1hour at 10°C. Concentrated aqueous HCl was then added until pH = 7. The resulting precipitate was filtered, washed with 1000 ml of water and air dried to yield 28.3 g of a yellow intermediate.

The 28.3 g of the obtained intermediate was taken up in 200 ml of methylethylketone, 47 g of methyliodide were added and the resulting mixture was refluxed for 48 hours. Temperature was cooled to room temperature and the formed precipitate was filtered, washed 3 times with each 15 ml of methylethylketone and dried under vacuum at 60°C for 24 hours. 22.4 g of compound of formula (a1) were obtained as an orange solid.

### Example 18 to 22

The preparation according to example 17 was done using the respective aniline compound and the respective alkylating agent to yield the compounds of formula (a2) to (a6). In case of examples 18 to 19 methyliodide and examples 20 to 22 allylbromide as alkylating agents were used. In the case of allylbromide as alkylating agent, if the desired final compound of formula (a4) to (a6) did not precipitate, the reaction mixture was evaporated to dryness and the compound used without further purification. The combinations and details are given in table (A7_1) and (A7_2).

| **TABLE (A7_1)** | | | | |
|---|---|---|---|---|
| | **Step 1: diazotation/copulation** | | | |
| **Ex.** | **1,3,3-trimethyl-2-methyl-indoline** | **Aniline compound** | | **Intermediate obtained** |
| | **[g]** | | **[g]** | **[g]** |
| **17** | 17.6 | 2-methoxyaniline | 12.4 | 28.3 |
| **18** | 44.4 | 2-aminophenyl-phenylsulfide | 51.8 | 87.2 |
| **19** | 12.7 | 4-isopropylaniline | 10.1 | 21.8 |
| **20** | 35.2 | 4-methoxyaniline | 25.2 | 57.8 |
| **21** | 44.4 | 2-aminophenyl-phenylsulfide | 51.8 | 87.2 |
| **22** | 17.6 | 2-methoxyaniline | 12.4 | 28.3 |

| **TABLE (A7_2)** | | | | | |
|---|---|---|---|---|---|
| | **Step 2 : alkylation** | | | | |
| **Ex.** | **Intermediate used** | **Alkylating agent** | | **Compound of formula** | |
| | **[g]** | | **[g]** | | **[g]** |
| **17** | 28.3 | Methyliodide | 47.0 | (a1) | 22.4 |
| **18** | 20.2 | Methyliodide | 22.6 | (a2) | 12.7 |
| **19** | 5.9 | Methyliodide | 8.0 | (a3) | 8.0 |
| **20** | 15.4 | Allylbromide | 37.0 | (a4) | 12.4 |
| **21** | 22.4 | Allylbromide | 42.1 | (a5) | 24.2 |
| **22** | 12.3 | Allylbromide | 14.5 | (a6) | 18.4 |

### Example 23

48.7 g of 2,3,3-trimethylindolenine and 108.9 g of allylbromide were refluxed in 150 ml of methylethylketone for 48 hours. After being cooled to room temperature the mixture was transfered into a separatory funnel and the brown bottom layer was separated. The crude 1-allyl-2,3,3-trimethylindolenium bromide was used for the next step without further purification.

9.06 g of conc. aqueous HCl were added dropwise to a solution composed of 3.77 g of 4-methoxyaniline in 50 ml of water. Temperature was decreased to 0°C with an ice bath and 6.2 ml of a solution of aqueous sodium nitrite (33.3% by weight) were added dropwise while temperature was maintained below 5°C. The resulting solution was stirred at 0°C for 1 hour and added dropwise to a mixture composed of 8.4 g of the crude 1-allyl-2,3,3-trimethylindolenium bromide, 14.8 g of AcONa and 50 ml of methanol.

After complete addition, the resulting mixture was stirred 1hour at room temperature. The solvent were removed under reduce pressure. To the remaining residue was added dichloromethane and the obtained mixture was filtered through celite. The filtrate was evaporated to dryness to afford 9.44 g of brown viscous oil.

The obtained oil was dissolved in 40 ml of methylethylketone, 12.04 g of methyliodide were added and the resulting mixture was refluxed for 24 hours. Additional 12.04 g of methyliodide were added and reflux was continued for 24 hours. After being cooled to room temperature, volatiles were removed under reduce pressure. The obtained residue containing compound of formula (a7) was used in the next step without further purification.

Table A8 shows the phys-chem properties of the compounds of formulae (a1) to (a7).

| **Table (A8)** | | | | |
|---|---|---|---|---|
| **Ex.** | **Compound of formula** | **λ max [nm]** | **ε (at λ max) [L / g * cm]** | **MP/ DP [°C]** |
| **17** | (a1) | 411 | 49 | 208(MP), 244 (DP) |
| **18** | (a2) | 403 | 60 | 207 (MP), 247 (DP) |
| **19** | (a3) | 445 | 72 | 262 (MP), 266 (DP) |
| **20** | (a4) | ### | ### | ### |
| **21** | (a5) | ### | ### | ### |
| **22** | (a6) | ### | ### | ### |
| **23** | (a7) | ### | ### | ### |

### Example 24

7.6 g of Sandocryl Golden Yellow C-GL p (purity 53%, C.I. Basic Yellow 28, abbreviation BY28) were refluxed for 1hour in 76 ml of ethanol. After being cooled to room temperature, the mixture was filtered. The filtrate was then added dropwise to a refluxing mixture composed of 4.9 g of compound of formula (e1) and 49 ml of acetonitrile. After complete addition, the mixture was refluxed for 4h. After being cooled to 10°C, the precipitate was filtered, washed with 30 ml of ethanol, 1000 ml of water and dried under vaccum at 65°C for 24 hours. 5.1 g of compound of formula (e1BY28) were obtained as an orange-brown solid.

### Example 25 to 41

The preparation according to example 24 was done using Basic Yellow 28 and the respective compounds of formulae (a1) to (a7) with regard to the compounds of formula (e1) to (e8) to yield the compounds of formula (e1a1), (e1a2), (e1a3), (e1a4), (e1a5), (e1a6), (e1a7), (e2BY28), (e2a2), (e3BY28), (e4BY28), (e4a2), (e5BY28), (e6BY28), (e6a2), (e7BY28) or (e8BY28).

When compounds of formula (a1) to (a7) were used, compounds of formula (a1) to (a7) and respective compounds of formula (e1) to (e8) were directly mixed and refluxed in a mixture of acetonitrile/ethanol without preliminary filtration.

In the case of compound of formula (e2), ethanol was used as sole solvent.

The combinations and details are given in table (A9).

| **Table (A9)** | | | | | | |
|---|---|---|---|---|---|---|
| **Ex.** | **Cobaltate complex** | | **Cation salt of formula** | | **compound of formula** | |
| | **Compound of formula** | **[g]** | | **[g]** | | **[g]** |
| **24** | (e1) | 4.9 | BY28 | 7.6 | (e1BY28) | 5.1 |
| **25** | (e1) | 5.0 | (a1) | 5.2 | (e1a1) | 4.4 |
| **26** | (e1) | 10.4 | (a2) | 9.4 | (e1a2) | 10.7 |
| **27** | (e1) | 4.3 | (a3) | 4.6 | (e1a3) | 5.3 |
| **28** | (e1) | 4.3 | (a4) | 4.7 | (e1a4) | 5.3 |
| **29** | (e1) | 5.4 | (a5) | 8.5 | (e1a5) | 6.2 |
| **30** | (e1) | ### | (a6) | ### | (e1a6) | ### |
| **31** | (e1) | 6.1 | (a7) | 6.7 | (e1a7) | 7.1 |
| **32** | (e2) | 5.1 | (BY28) | 2.6 | (e2BY28) | 4.7 |
| **33** | (e2) | 2.3 | (a2) | 1.2 | (e2a2) | 2.1 |
| **34** | (e3) | 5.0 | (BY28) | 7.0 | (e3BY28) | 6.1 |
| **35** | (e4) | 4.0 | (BY28) | 5.5 | (e4BY28) | 3.0 |
| **36** | (e4) | 4.0 | (a2) | 3.2 | (e4a2) | 2.8 |
| **37** | (e5) | 2.9 | (BY28) | 2.4 | (e5BY28) | ### |
| **38** | (e6) | 10.0 | (BY28) | 14.9 | (e6BY28) | 11.8 |
| **39** | (e6) | 5.0 | (a2) | 4.4 | (e6a2) | 5.5 |
| **40** | (e7) | 7.1 | (BY28) | 10.2 | (e7BY28) | 7.6 |
| **41** | (e8) | 9.2 | (BY28) | 13.5 | (e8BY28) | 10.0 |

Table A10 shows the phys-chem properties of the compounds resulting of examples (24) to (41).

| **Table (A10)** | | | | |
|---|---|---|---|---|
| **Ex.** | **Compound of formula** | **λ max [nm]** | **ε (at λ max) [L / g * cm]** | **MP/ DP [°C]** |
| **24** | (e1BY28) | 480 | 84 | 316 (DP) |
| **25** | (e1a1) | 482 | 54 | 277 (DP) |
| **26** | (e1a2) | 483 | 64 | 285 (DP) |
| **27** | (e1a3) | 477 | 84 | 287 (DP) |
| **28** | (e1a4) | 480 | 70 | 251 (DP) |
| **29** | (e1a5) | 482 | 57 | 266 (DP) |
| **30** | (e1a6) | ### | ### | ### (DP) |
| **31** | (e1a7) | 481 | 74 | 260 (DP) |
| **32** | (e2BY28) | 481 | 67 | 231 (DP) |
| **33** | (e2a2) | 486 | 55 | 243 (DP) |
| **34** | (e3BY28) | 480 | 70 | 279 (DP) |
| **35** | (e4BY28) | 482 | 62 | 277 (DP) |
| **36** | (e4a2) | 404 | 49 | 280 (DP) |
| **37** | (e5BY28) | ### | ### | ### (DP) |
| **38** | (e6BY28) | 479 | 85 | 299 (DP) |
| **39** | (e6a2) | ### | ### | 277 (DP) |
| **40** | (e7BY28) | 479 | 78 | 276 (DP) |
| **41** | (e8BY28) | 479 | 84 | 276 (DP) |

### Application Example 1

The optical and thermal properties of the compounds of formula (I) were studied. The compounds of formula (I) show high absorption at the desired wavelengths. In addition, the shapes of the absorption spectra, that still remain critical to the disc reflectivity and formation of clean mark edges, are composed of one major band, comprised in a range of from 330 to 500 nm.

More precisely, n values of the refractive index were evaluated between 1.0 and 2.7. Light stabilities were found comparable to commercial dyes which are already stabilized with quenchers for the use in optical data recording.

Sharp threshold of thermal decomposition within the required temperature range characterizes the compounds of formula (I) which are desirable for the application in optical layers for optical data recording.

### Application Example 2 - Optical layer and optical data recording medium

1.4% by weight, based on the weight of the solvent, of the compound of formula (e1BY28) are dissolved in 2,2,3,3-tetrafluoropropan-1-ol and the solution is filtered through a Teflon filter of pore size 0.2 µm and applied by spin-coating at 1000 rpm to the surface of a 0.6 mm thick, grooved polycarbonate disc of 120 mm diameter. The excess solution is spun off by increasing the rotational speed. On evaporation of the solvent, the dye remains behind in the form of a uniform, amorphous solid layer, the optical layer.

After drying the optical layer in a circulating-air oven at 70°C (10 min) in a vacuum coating apparatus, a 100 µm thick silver layer is then applied to the recording layer by atomization. Then a 6 µm thick protective layer of a UV curable photopolymer (650-020, DSM) is applied thereto by means of spincoating. Finally, a second substrate is provided to combine with the resin protection layer using an attachment layer. This completes the manufacturing of a high-density recordable optical disc, the optical data recording medium.

Evaluation tests are performed using an optical disk evaluation device available from Pulse Tech Co., Ltd.

The testing conditions are the following ones:
- Numerical aperture (NA) of the optical head: 0.65
- Wavelength of a laser light for recording and reproduction: 405 nm
- Constant linear velocity (CLV): 6.61 m/sec.
- Track pitch: 400 nm
- Wobble amplitude of the groove track: 14 nm
- Groove depth: 90 nm.

### Comparative application example 1

Application example 2 was carried out using only the compound of formula (d6BY28).

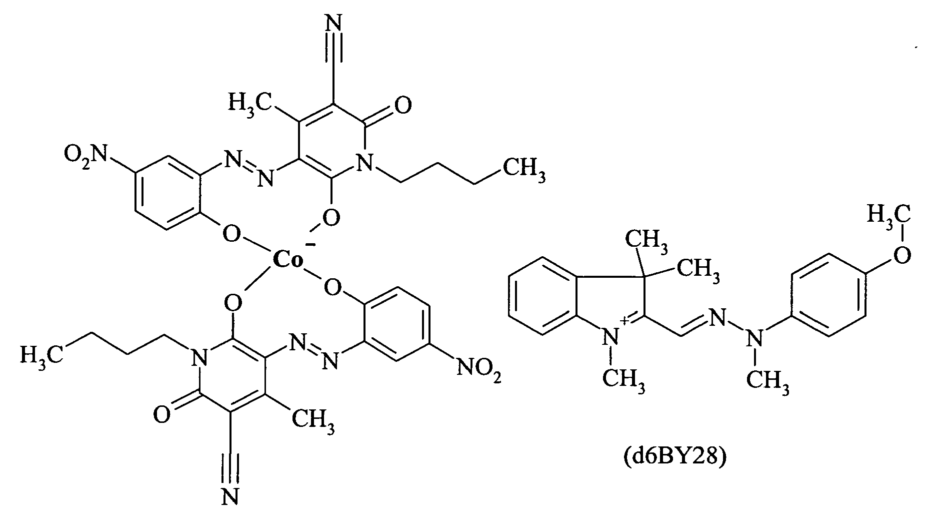

Results of the testing in analogy to application example 2 with various compounds of formula (I) are summarized in the table (D). The compounds of formula (I) were prepared according to the examples as described.

| **Table (D)** | | | | | | |
|---|---|---|---|---|---|---|
| | | | | | | |
| **Ex.** | **Compound of formula** | **Pw (mW)** | **SbER** | **PRSNR** | **modulation** | **reflectivity [%]** |
| | | | | | | |
| | | **Comparative application example 1** | | | | |
| | (d6BY28) | 8 | 3.2*10E-06 | 22.3 | 0.49 | 19.4 |

| **Application example 2 to 19** | | | | | | |
|---|---|---|---|---|---|---|
| **2** | (e1BY28) | 6.8 | 3.6*10E-09 | 35.3 | 0.61 | 18.3 |
| **3** | (e1a1) | ### | ### | ### | ### | ### |
| **4** | (e1a2) | 7.0 | 6.0*10E-09 | 32.0 | 0.56 | 16.5 |
| **5** | (e1a3) | 6.4 | 3.3*10E-10 | 31.0 | 0.60 | 17.3 |
| **6** | (e1a4) | 6.5 | 2.0*10E-09 | 31.1 | 0.57 | 18.4 |
| **7** | (e1a5) | 6.8 | 5.8*10E-10 | 33.4 | 0.56 | 17.0 |
| **8** | (e1a6) | ### | ### | ### | ### | ### |
| **9** | (e1a7) | ### | ### | ### | ### | ### |
| **10** | (e2BY28) | 7.1 | 5.2*10E-09 | 33.2 | 0.54 | 18.5 |
| **11** | (e2a2) | ### | ### | ### | ### | ### |
| **12** | (e3BY28) | 7.1 | 1.7*10E-09 | 31.9 | 0.51 | 19.7 |
| **13** | (e4BY28) | ### | ### | ### | ### | ### |
| **14** | (e4a2) | ### | ### | ### | ### | ### |
| **15** | (e5BY28) | ### | ### | ### | ### | ### |
| **16** | (e6BY28) | ### | ### | ### | ### | ### |
| **17** | (e6a2) | ### | ### | ### | ### | ### |
| **18** | (e7BY28) | ### | ### | ### | ### | ### |
| **19** | (e8BY28) | ### | ### | ### | ### | ### |

A test for evaluating a degree of degradation due to repetition reproduction is conducted for each of the write-once optical disks made for the described recording layers. Readings are carried out at a reading laser power of 0.4 mW and the degrees of degradation of PRSNR and SbER are then measured. Maximum cycle number was found within the specifications.

## Claims

1. A compound of formula (I), 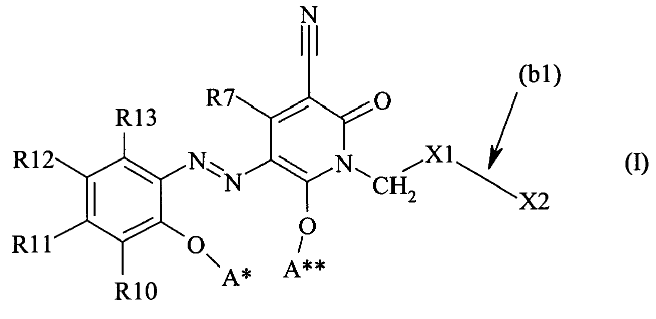 wherein the residues A* and A**each represent H
or the residues A* and A** together represent a group of formula (II); 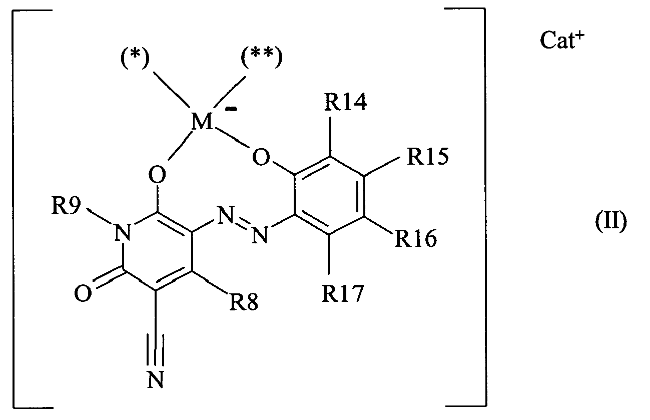 M represents a trivalent metal atom;
the (*) in formula (II) indicating the bond from the metal atom M to the O atom, which is connected to the residue A* in formula (I), and
the (**) in formula (II) indicating the bond from the metal atom M to the O atom, which is connected to the residue A** in formula (I);
X1 represents a carbon atom in the case, that the bond (b1) to X2 is a triple bond, or
X1 represents -CH- in the case, that the bond (b1) to X2 is a double bond;
X2 represents a N atom or CH in the case, that the bond (b1) to X2 is a triple bond, or
X2 represents CH2 in the case, that the bond (b1) to X2 is a double bond; or
X1 together with X2 form a phenyl group and the bond (b1) is an aromatic bond;
Cat⁺ is a cation selected from the group consisting of basic yellow cations of Basic Yellow dyes, compounds of formula (a) and compounds of formula (g); 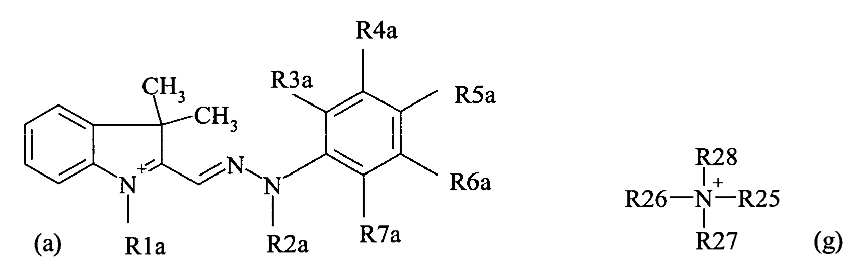
R9 is selected from the group consisting of -C₁₋₄ alkyl, -NH-phenyl, -CH₂-CH=CH₂, -CH₂-C≡CH, -CH₂-CN and benzyl;
R7 and R8 are identical or different and independently from each other selected from the group consisting of H, CN, CF₃, halogen, NO₂, OH, SH, SO₂-NR²¹R²², CO-R²⁰, SO₂R²⁰, CO-NR²¹R²²,
C₁₋₁₀ alkyl, C₃₋₁₀ cycloalkyl, the C₁₋₁₀ alkyl and the C₃₋₁₀ cycloalkyl being independently from each other unsubstituted or substituted by 1 to 4 identical or different substituents, the substituents being independently from each other selected from the group consisting of C₁₋₁₀ alkyl, halogen, OH, C₆₋₁₂ aryl and NR²¹R²²,
C₆-C₁₂ aryl the C₆₋₁₂ aryl being unsubstituted or substituted by 1 to 4 identical or different substituents; the substituents being independently from each other selected from the group consisting of C₁₋₁₀ alkyl, C₃₋₁₀ cycloalkyl, OH, NO₂, CN, halogen, CF₃, C₆₋₁₂ aryl, C₁₋₁₀ alkoxy and NR²¹R²²,
OC₁₋₁₀ alkyl, NR²¹R²² and SC₁₋₁₀ alkyl;
R3a, R4a, R5a, R6a, R7a, R10, R11, R12, R13, R14, R15, R16 and R17 are identical or different and independently from each other selected from the group consisting ofH, CN, CF₃, halogen, NO₂, OH, SH, SO₂-NR²¹R²², CO-R²⁰, SO₂R²⁰, CO-NR²¹R²²,
C₁₋₁₀ alkyl, C₃₋₁₀ cycloalkyl, the C₁₋₁₀ alkyl and the C₃₋₁₀ cycloalkyl being independently from each other unsubstituted or substituted by 1 to 4 identical or different substituents, the substituents being independently from each other selected from the group consisting of C₁₋₁₀ alkyl, halogen, OH, CN, CF₃, C₆₋₁₂ aryl and NR²¹R²²,
C₆-C₁₂ aryl, O-C₆₋₁₂ aryl, S-C₆₋₁₂ aryl, the C₆₋₁₂ aryl and the O-C₆₋₁₂ aryl and the S-C₆₋₁₂ aryl being unsubstituted or substituted by 1 to 4 identical or different substituents, the substituents being independently from each other selected from the group consisting of C₁₋₁₀ alkyl, C₃₋₁₀ cycloalkyl, OH, NO₂, CN, halogen, CF₃, C₆₋₁₂ aryl, O-C₁₋₁₀ alkyl, S-C₁₋₁₀ alkyl and NR²¹R²²,
OC₁₋₁₀ alkyl, SC₁₋₁₀ alkyl, O-C₃₋₁₀ cycloalkyl, S-C₃₋₁₀ cycloalkyl, NHCOR²⁰ and NR²¹R²²;
the R²¹ and R²² residues are identical or different and independently from each other selected from the group consisting of H, C₁₋₁₀ alkyl, C₆₋₁₂ aryl and C₁₋₁₂ alkyl-NR²³R²⁴;
the R²³ and R²⁴ residues are identical or different and independently from each other selected from the group consisting of H, C₁₋₁₀ alkyl and C₆₋₁₂ aryl;
the R²⁰ residues are identical or different and independently from each other selected from the group consisting of OH, C₁₋₆ alkyl, C₆₋₁₀ aryl and O-C₁₋₆ alkyl;
R1a and R2a are identical or different and independently from each other selected from the group consisting of
C₁₋₁₀ alkyl, C₂₋₁₀ alkenyl, C₂₋₁₀ alkynyl, C₃₋₁₀ cycloalkyl,
the C₁₋₁₀ alkyl, the C₂₋₁₀ alkenyl, the C₂₋₁₀ alkynyl and the C₃₋₁₀ cycloalkyl being independently from each other unsubstituted or substituted by identical or different substituents, preferably by 1 or more than 1 substituent, more preferably by 1, 2, 3 or 4 substituents, even more preferably by 1 substituent, the substituents being independently from each other selected from the group consisting of C₁₋₁₀ alkyl, halogen, CN, OH, CF₃, C₆₋₁₂ aryl, the C₆₋₁₂ aryl being unsubstituted or substituted by 1 to 5, preferably 1 to 3, more preferably 1 or 2, identical or different substituents, the substituents being independently from each other selected from the group consisting of C₁₋₁₀ alkyl, OH, NO₂, CN, halogen, CF₃, C₆₋₁₂ aryl, O-C₁₋₁₀ alkyl and S-C₁₋₁₀ alkyl;
the C₂₋₁₀ alkenyl having 1, 2 or 3, preferably 1 or 2, more preferably 1 double bond, the double bond being preferably a beta-gamma-double bond, more preferably the C₂₋₁₀ alkenyl represents allyl;
the C₂₋₁₀ alkynyl having 1, 2 or 3, preferably 1 or 2, more preferably 1 triple bond, the triple bond being preferably a beta-gamma-triple bond, more preferably the C₂₋₁₀ alkynyl represents propargyl;
the compounds of formula (g) being also called ammonium type cations of formula (g); R²⁵, R²⁶, R²¹ and R²⁸ are identical or different and independently from each other selected from the group consisting of H, C₁₋₃₀ aliphatic hydrocarbon and C₁₋₃₀ aromatic hydrocarbon, the C₁₋₃₀ aliphatic and/or C₁₋₃₀ aromatic hydrocarbon being unsubstituted or substituted by 1 to 8 identical or different substituents independently from each other selected from the group consisting of halogen, C₁₋₁₀-alkyl, O-C₁₋₁₀-alkyl, CN, NH₂, OH, NO₂;
or where the substituents
R²⁵ and R²⁶ together with the nitrogen atom of the ammonium ion of formula (g) to which they are attached
can form a five- to seven-membered saturated or unsaturated ring which can contain 1, 2 or 3, preferably 1 or 2 further identical or different heteroatoms independently from each other selected from the group consisting of O, S and N or carbonyl groups,
and which can carry 1 or 2 fused-on saturated, unsaturated or aromatic, carbocyclic or heterocyclic rings;
the ring and the rings fused on, where appropriate, can be substituted by 1, 2 or 3 identical or different radicals independently from each other selected from the group consisting of OH, NH₂, phenyl, CN, Cl, Br, C₁-C₄ alkyl and C₁-C₄ alkoxy,
preferably of the pyrrolidine, pyrrolidone, imidazolidine, hexamethyleneimine, piperidine, piperazine or morpholine type, more preferably of the pyrrolidine or morpholine type;
or where the substituents
R²⁵, R²⁶ and R²⁷ together with the nitrogen atom of the ammonium ion of formula (g) to which they are attached
can form a five- to seven-membered aromatic ring which can contain 1, 2 or 3, preferably 1 or 2 further identical or different heteroatoms independently from each other selected from the group O, S and N or carbonyl groups,
and which can carry 1 or 2 fused-on saturated, unsaturated or aromatic, carbocyclic or heterocyclic rings;
the ring and the rings fused on, where appropriate, can be substituted by 1, 2 or 3 identical or different radicals independently from each other selected from the group consisting of OH, NH₂, phenyl, CN, Cl, Br, C₁-C₄ alkyl and C₁-C₄ alkoxy,
preferably of the pyrrole, imidazole, pyridine, picoline, pyrazine, quinoline or isoquinoline type.

2. A process for the preparation of a compound of formula (I) with A* and A** each being H as defined in claim 1, by an azo coupling reaction of the respective compound of formula (Ia) with the respective compound of formula (Ib), 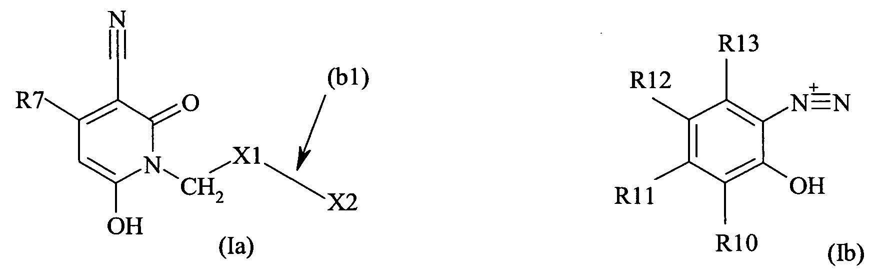 wherein (bl), R7, R10, R11, R12, R13, X1 and X2 are defined as in claim 1.

3. A process for the preparation of a compound of formula (I) with the residues A* and A** together representing a group of formula (II) as defined in claim 1, by a complexing reaction of a compound of formula (I) with A* and A** being H as defined in claim 1 and a compound of formula (III), 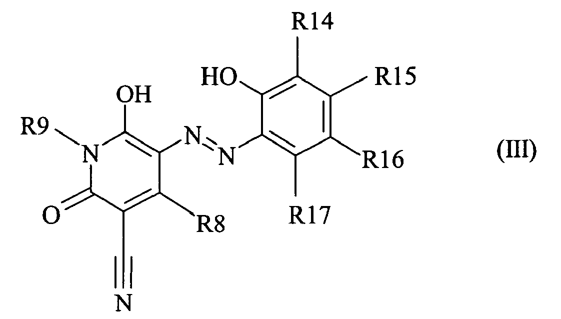 with R8, R9, R14, R15, R16 and R17 being as defined in claim 1,
with a metal salt, in the presence of Cat⁺ or with in situ formation of Cat+;
or by metathesis of respective precursor salts.

4. A compound of formula (Ia) as defined in claim 2.

5. Use of a compound of formula (Ia) as defined in claim 4, for the preparation of a compound of formula (I) as defined in claim 1.

6. Use of a compound of formula (I) with A* and A** each being H as defined in claim 1 as a ligand in azo metal complex dyes.

7. Use of a compound of formula (I) as defined in claim 1 in an optical layer.

8. Use of a compound of formula (I) as defined in claim 1 as a dye in an optical layer.

9. An optical layer comprising a compound of formula (I) as defined in claim 1.

10. A method for producing an optical layer as defined in claim 9, comprising the following steps
(a) providing a substrate,
(b) dissolving at least one compound of formula (I) as defined in claim 1 in an organic solvent to form a solution,
(c) coating the solution (b) on the substrate (a),
(d) evaporating the solvent to form an optical layer.

11. An optical data recording medium comprising an optical layer as defined in claim 9.
